# EUROPEAN PATENT SPECIFICATION

(11) **EP 3 227 445 B1**
(45) Date of publication and mention of the grant of the patent: **14.10.2020**
(21) Application number: 15807980.6
(22) Date of filing: 01.12.2015
(51) Int. Cl.: C12N 15/113

(54) **CELL PENETRATING MOLECULE**
ZELLENPENETRIERENDES MOLEKÜL
MOLÉCULE DE PÉNÉTRATION CELLULAIRE

(30) Priority: 02.12.2014 GB 201421379
(43) Date of publication of application: 11.10.2017
(73) Proprietor: Oxford University Innovation Limited, Oxford OX2 0SZ (GB); United Kingdom Research and Innovation, Swindon SN2 1FL (GB)
(72) Inventor: WOOD, Matthew, Oxford Oxfordshire OX1 3QX (GB); MCCLOREY, Graham, Oxford Oxfordshire OX1 3QX (GB); GAIT, Michael, Cambridge Cambridgeshire CB2 0QH (GB); JARVER, Peter, Cambridge Cambridgeshire CB2 0QH (GB); SALAH, Amer, Cambridge Cambridgeshire CB2 0QH (GB); SHABANPOOR, Fazel, Cambridge Cambridgeshire CB2 0QH (GB)
(74) Representative: J A Kemp LLP
(86) International application number: PCT/GB2015/053667
(87) International publication number: WO 2016/087842

(56) References cited:
- WO-A1-2013/040429
- WO-A1-2014/043544
- WO-A1-2015/113922
- WO-A2-2008/109105

### SEQUENCE LISTING

<110> ISIS INNOVATION LIMITED MEDICAL RESEARCH COUNCIL
<120> CELL PENETRATING MOLECULE
<130> N404322WO
<150> GB 1421379.7
   <151> 2014-12-02
<160> 18
<170> PatentIn version 3.5
<210> 1
   <211> 22
   <212> PRT
   <213> Artificial
<220>
   <223> cell penetrating peptide sequence
<220>
   <221> misc_feature
   <222> (2)..(2)
   <223> Xaa is 6-aminohexanoic acid
<220>
   <221> misc_feature
   <222> (5)..(5)
   <223> Xaa is bAla
<220>
   <221> misc_feature
   <222> (8)..(8)
   <223> Xaa is 6-aminohexanoic acid
<220>
   <221> misc_feature
   <222> (16)..(16)
   <223> Xaa is 6-aminohexanoic acid
<220>
   <221> misc_feature
   <222> (18)..(18)
   <223> Xaa is bAla
<220>
   <221> misc_feature
   <222> (20)..(20)
   <223> Xaa is 6-aminohexanoic acid
<220>
   <221> misc_feature
   <222> (22)..(22)
   <223> Xaa is bAla
<400> 1
   Arg Xaa Arg Arg Xaa Arg Arg Xaa Arg Tyr Gln Phe Leu Ile Arg Xaa
   1               5                   10                  15
   Arg Xaa Arg Xaa Arg Xaa
               20
<210> 2
   <211> 14
   <212> PRT
   <213> Artificial
<220>
   <223> Cell penetrating peptide sequence
<220>
   <221> misc_feature
   <222> (2)..(2)
   <223> Xaa is 6-aminohexanoic acid
<220>
   <221> misc_feature
   <222> (5)..(5)
   <223> Xaa is bAla
<220>
   <221> misc_feature
   <222> (8)..(8)
   <223> Xaa is 6-aminohexanoic acid
<220>
   <221> misc_feature
   <222> (11)..(11)
   <223> Xaa is bAla
<220>
   <221> misc_feature
   <222> (13)..(13)
   <223> Xaa is 6-aminohexanoic acid
<220>
   <221> misc_feature
   <222> (14)..(14)
   <223> Xaa is bAla
<400> 2
   Arg Xaa Arg Arg Xaa Arg Arg Xaa Arg Arg Xaa Arg Xaa Xaa
   1               5                   10
<210> 3
   <211> 13
   <212> PRT
   <213> Artificial
<220>
   <223> Cell penetrating peptide sequence
<400> 3
   Gly Arg Lys Lys Arg Arg Gln Arg Arg Arg Pro Pro Gln
   1               5                   10
<210> 4
   <211> 27
   <212> PRT
   <213> Artificial
<220>
   <223> Cell penetrating peptide sequence
<400> 4
   Gly Trp Thr Leu Asn Ser Ala Gly Tyr Leu Leu Gly Lys Ile Asn Leu
   1               5                   10                  15
   Lys Ala Leu Ala Ala Leu Ala Lys Lys Ile Leu
               20                  25
<210> 5
   <211> 16
   <212> PRT
   <213> Artificial
<220>
   <223> Cell penetrating peptide sequence
<400> 5
   Arg Gln Ile Lys Ile Trp Phe Gln Asn Arg Arg Met Lys Trp Lys Lys
   1               5                   10                  15
<210> 6
   <211> 22
   <212> PRT
   <213> Artificial
<220>
   <223> Cell penetrating peptide sequence
<400> 6
   Arg Arg Arg Arg Arg Arg Arg Gln Ile Lys Ile Trp Phe Gln Asn Arg
   1               5                   10                  15
   Arg Met Lys Trp Lys Lys
               20
<210> 7
   <211> 18
   <212> PRT
   <213> Artificial
<220>
   <223> Cell penetrating peptide sequence
<400> 7
   Leu Leu Ile Ile Leu Arg Arg Arg Ile Arg Lys Gln Ala His Ala His
   1               5                   10                  15
   Ser Lys
<210> 8
   <211> 27
   <212> PRT
   <213> Artificial
<220>
   <223> Cell penetrating peptide sequence
<400> 8
   Gly Ala Leu Phe Leu Gly Phe Leu Gly Ala Ala Gly Ser Thr Met Gly
   1               5                   10                  15
   Ala Trp Ser Gln Pro Lys Lys Lys Arg Lys Val
               20                  25
<210> 9
   <211> 21
   <212> PRT
   <213> Artificial
<220>
   <223> Cell penetrating peptide sequence
<400> 9
   Lys Glu Thr Trp Trp Glu Thr Trp Trp Thr Glu Trp Ser Gln Pro Lys
   1               5                   10                  15
   Lys Lys Arg Lys Val
               20
<210> 10
   <211> 18
   <212> PRT
   <213> Artificial
<220>
   <223> Cell penetrating peptide sequence
<400> 10
   Lys Leu Ala Leu Lys Leu Ala Leu Lys Ala Leu Lys Ala Ala Leu Lys
   1               5                   10                  15
   Leu Ala
<210> 11
   <211> 9
   <212> PRT
   <213> Artificial
<220>
   <223> Cell penetrating peptide sequence
<400> 11
   Arg Arg Trp Trp Arg Arg Trp Arg Arg
   1 5
<210> 12
   <211> 42
   <212> PRT
   <213> Artificial
<220>
   <223> Cell penetrating peptide sequence
<400> 12
   Tyr Thr Ile Trp Met Pro Glu Asn Pro Arg Pro Gly Thr Pro Cys Asp
   1 5 10 15
   Ile Phe Thr Asn Ser Arg Gly Lys Arg Ala Ser Asn Gly Gly Gly Gly
   20 25 30
   Arg Arg Arg Arg Arg Arg Arg Arg Arg Arg
   35 40
<210> 13
   <211> 20
   <212> DNA
   <213> Homo sapiens
<400> 13
   ggccaaacct cggcttacct 20
<210> 14
   <211> 25
   <212> DNA
   <213> Homo sapiens
<400> 14
   ggccaaacct cggcttacct gaaat 25
<210> 15
   <211> 25
   <212> DNA
   <213> Homo sapiens
<400> 15
   gcctcgtttc tcggcagcaa tgaac 25
<210> 16
   <211> 22
   <212> PRT
   <213> Artificial
<220>
   <223> Cell penetrating peptide sequence
<220>
   <221> misc_feature
   <222> (2)..(2)
   <223> Xaa is 6-aminohexanoic acid
<220>
   <221> misc_feature
   <222> (5)..(5)
   <223> Xaa is bAla
<220>
   <221> misc_feature
   <222> (8)..(8)
   <223> Xaa is 6-aminohexanoic acid
<220>
   <221> misc_feature
   <222> (16)..(16)
   <223> Xaa is 6-aminohexanoic acid
<220>
   <221> misc_feature
   <222> (18)..(18)
   <223> Xaa is bAla
<220>
   <221> misc_feature
   <222> (20)..(20)
   <223> Xaa is 6-aminohexanoic acid
<220>
   <221> misc_feature
   <222> (22)..(22)
   <223> Xaa is bAla
<400> 16
   Arg Xaa Arg Arg Xaa Arg Arg Xaa Arg Ile Leu Phe Gln Tyr Arg Xaa
   1               5                   10                  15
   Arg Xaa Arg Xaa Arg Xaa
               20
<210> 17
   <211> 15
   <212> DNA
   <213> Homo sapiens
<400> 17
   cuuucauaau gcugg 15
<210> 18
   <211> 15
   <212> DNA
   <213> Homo sapiens
<400> 18
   ccagcauuau gaaag 15

## Description

### Field of the Invention

The present invention relates to cell penetrating molecules comprising two oligonucleotide cargo molecules.

### Background to the Invention

Antisense and RNAi oligonucleotides show promise as therapeutic agents, but typically exhibit only poor delivery to and/or uptake into cells, which limits their therapeutic efficacy. Various strategies have been used to try to improve cellular delivery and/or uptake of such oligonucleotides to address this problem. One such strategy involves use of a delivery agent, to which the oligonucleotide is attached as a cargo molecule. Unfortunately though such an approach can itself cause problems. For example, the delivery agent may demonstrate relatively high levels of cellular toxicity which can restrict their utility in a therapeutic setting.

WO2013/040429 and WO2014/043544 both disclose multimeric oligonucleotide compounds. WO2008/109105 discloses methods and compositions for improved therapeutic effects with siRNA.

### Summary of the invention

The present inventors have made the surprising discovery that, in certain specific arrangements, it is possible to attach particular combinations of two oligonucleotide cargo molecules to a cell penetrating peptide (CPP) without eliminating the cell delivery capability of the CPP and such that both oligonucleotides exhibit biological activity in a cell to which they are delivered. The oligonucleotide cargo molecules are typically antisense or RNAi oligonucleotides.

In one embodiment, the two oligonucleotides may be identical to each other or may specifically hybridise to the same target sequence in a cell. This carries the advantage that the CPP carrying the two oligonucleotides can result in the same level of oligonucleotide anti-target activity in a cell (or a patient) at a lower concentration of CPP, as compared to a CPP carrying only a single oligonucleotide cargo molecule. Thus any toxicity associated with the CPP will be reduced.

In another embodiment, each of the two oligonucleotides may specifically hybridise to a different target sequence within the same RNA molecule. Alternatively, each of the oligonucleotides may specifically hybridise to a target sequence within a different RNA molecule, but said RNA molecules are encoded by sequences in the genome of a cell that at least partially overlap with each other on the same or the opposite DNA strand. Thus in these two embodiments a single molecule of the invention targets two different sequences in the same RNA molecule, or targets two closely-related RNA molecules. This may be advantageous in some diseases or conditions, in which the same overall presentation of symptoms may arise in different sub-groups of patients as a result of different defects in the same RNA molecule or in closely-related RNA molecules. The single molecule of the invention in these two embodiments is capable of treating multiple patient sub-groups suffering from such a disease or condition, whereas a CPP carrying a single oligonucleotide cargo molecule can treat only a single sub-group.

Thus, the present invention provides a molecule having the structure of formula (I):

(CPP)-(S1)-(L1)-(S2)-(L2);

wherein:
(a)
   (CPP) is a cell penetrating peptide arranged in the N to C terminal direction;
   (S1) and (S2) are spacer moieties which are independently present or absent;
   (L1) is a first linking moiety;
   (L2) is a second linking moiety;
(b) each of (L1) and (L2) is independently attached to a separate cargo molecule which comprises or consists of an oligonucleotide;
(c) each said oligonucleotide comprises a sequence which specifically hybridises to a target sequence and either:
   (i) the oligonucleotide attached to (L1) is identical to the oligonucleotide attached to (L2); or
   (ii) the oligonucleotide attached to (L1) and the oligonucleotide attached to (L2) each comprise a sequence which specifically hybridises to the same target sequence; or
   (iii) the oligonucleotide attached to (L1) comprises a sequence which specifically hybridises to a first target sequence in an RNA molecule and the oligonucleotide attached to (L2) comprises a sequence which specifically hybridises to a second, different target sequence in the same RNA molecule; or
   (iv) the oligonucleotide attached to (L1) comprises a sequence which specifically hybridises to a first target sequence in an RNA molecule, the sequence of which RNA molecule is encoded by a sequence in the genome of a cell which at least partially overlaps on the same or the opposite DNA strand with a sequence in said genome which encodes a second RNA molecule, and the oligonucleotide attached to (L2) comprises a sequence which specifically hybridises to a target sequence in said second RNA molecule.

Also provided is a molecule of formula (II):

(L1)-(S1)-(CPP)-(S2)-(L2);

wherein:
(a)
   (CPP) is a cell penetrating peptide arranged in the N to C terminal direction;
   (S1) and (S2) are spacer moieties which are independently present or absent;
   (L1) is a first linking moiety;
   (L2) is a second linking moiety;
(b) each of (L1) and (L2) is independently attached to a separate cargo molecule which comprises or consists of an oligonucleotide;
(c) each said oligonucleotide comprises a sequence which specifically hybridises to a target sequence and either:
   (i) the oligonucleotide attached to (L1) is identical to the oligonucleotide attached to (L2); or
   (ii) the oligonucleotide attached to (L1) and the oligonucleotide attached to (L2) each comprise a sequence which specifically hybridises to the same target sequence; or
   (iii) the oligonucleotide attached to (L1) comprises a sequence which specifically hybridises to a first target sequence in an RNA molecule and the oligonucleotide attached to (L2) comprises a sequence which specifically hybridises to a second, different target sequence in the same RNA molecule; or
   (iv) the oligonucleotide attached to (L1) comprises a sequence which specifically hybridises to a first target sequence in an RNA molecule, the sequence of which RNA molecule is encoded by a sequence in the genome of a cell which at least partially overlaps on the same or the opposite DNA strand with a sequence in said genome which encodes a second RNA molecule, and the oligonucleotide attached to (L2) comprises a sequence which specifically hybridises to a target sequence in said second RNA molecule.

Also provided is a pharmaceutical composition comprising a molecule of the invention and optionally a pharmaceutically acceptable diluent, adjuvant or carrier.

Also provided is a molecule or composition of the invention for use in a method of treatment of a disease or condition.

Also provided is the use a molecule or composition of the invention in the manufacture of a medicament for use in the treatment of a disease or condition.

Also provided is a method of treating a disease or condition in a patient comprising administering to the patient a therapeutically effective amount of a molecule or composition of the invention.

### Brief Description of the Sequence Listing

SEQ ID NOs: 1 to 12 and 16 are examples of CPP that may be used in a molecule of the invention.
SEQ ID NOs: 13 to 15 and 17 are examples of oligonucleotide cargo molecules that may be used in a molecule of the invention.
SEQ ID NO: 18 is an example of a sequence specifically hybridised by an exemplary oligonucleotide cargo molecule.

### Brief Description of the Figures

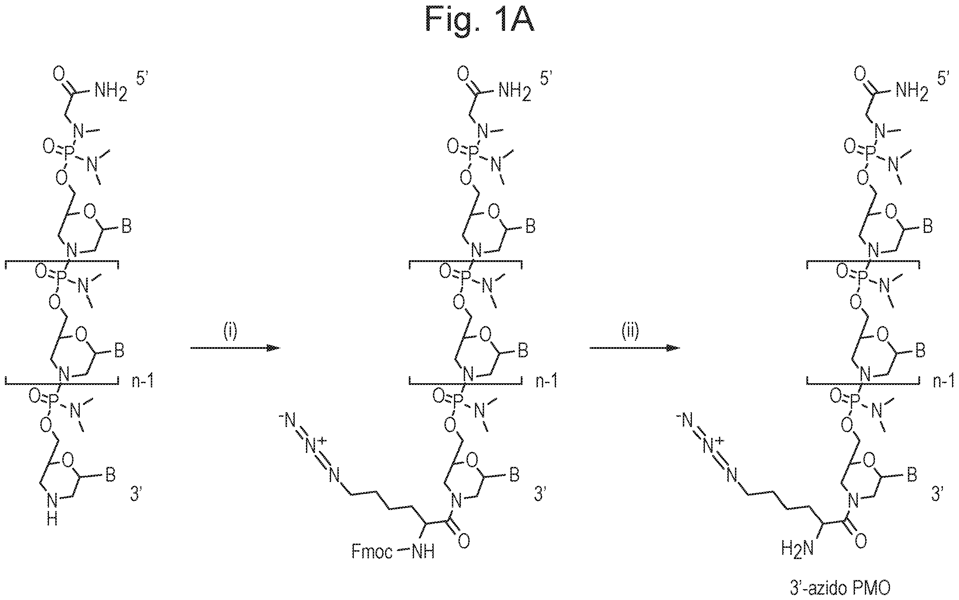

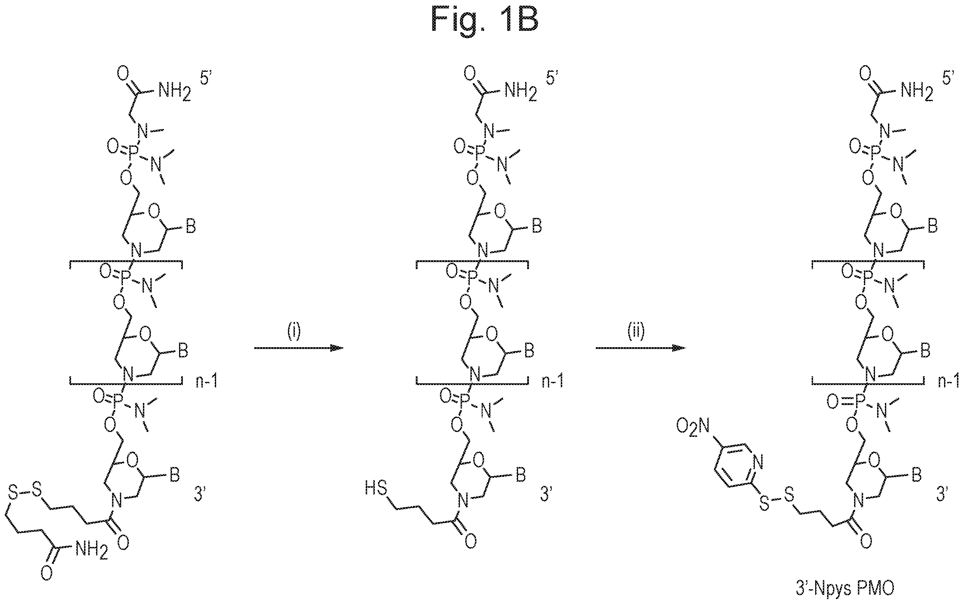

**Figure 1****.** Functionalization of a phosphorodiamidate morpholino oligonucleotide (PMO): **A** (i) conjugation of Fmoc-azido-L-Lysine-OH to the 3'-end of unmodified PMO and (ii) removal of the Fmoc group using 20% piperidine. **B** (i) reduction of the disulphide bond of 3'-disulphide functionalized PMO by treatment with a 10 fold excess of TCEP and (ii) activation of the resultant free thiol group using a 2.5-fold molar excess of 2,2'-dithiobis(5-nitropyridine) (DTNP) in DMSO: acetonitrile (0.1% TFA): H₂O (0.1% TFA) with (1:1:3) ratios.

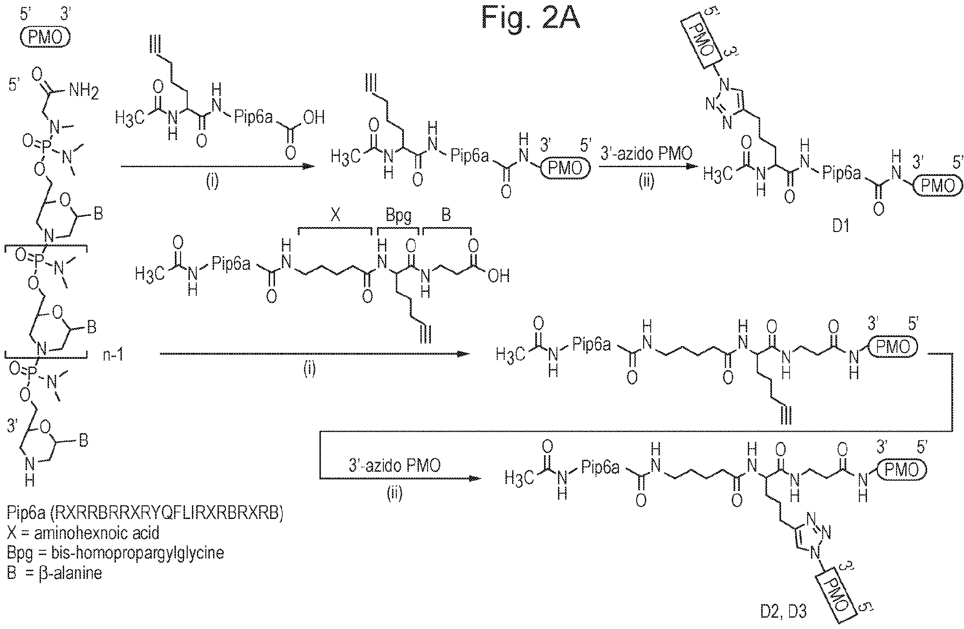

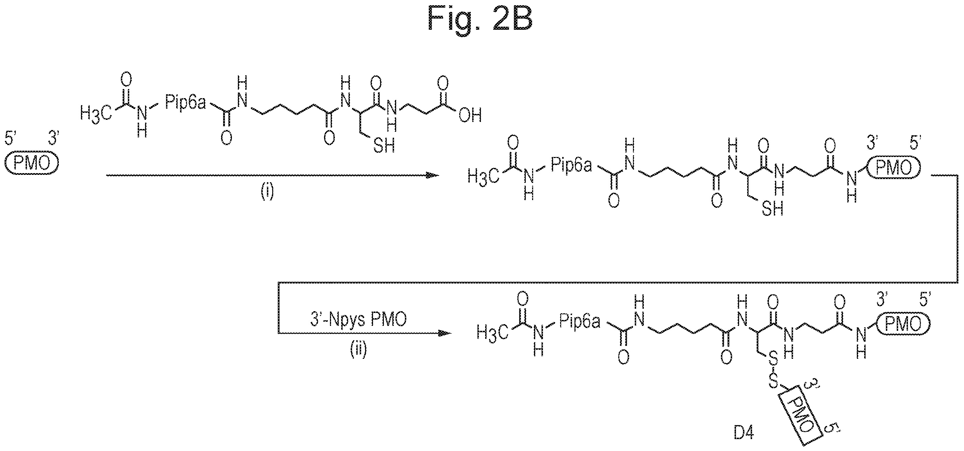

**Figure 2****. A** and **B** (i) conjugation of a first PMO to the C-terminal carboxylic acid of Pip6a peptide is effected through an amide bond. **A** (ii) alkyne-modified Pip6a-PMO is conjugated through alkyne-azide click chemistry to an azido second PMO using copper (I) to give conjugate D1 (one PMO at each terminus of Pip6a) or D2 and D3 (both PMOs at the C-terminus of Pip6a). **B** (ii) cysteine-modified Pip6a-PMO is conjugated through disulphide bond formation to the thiol-activated second PMO to give conjugate D4.

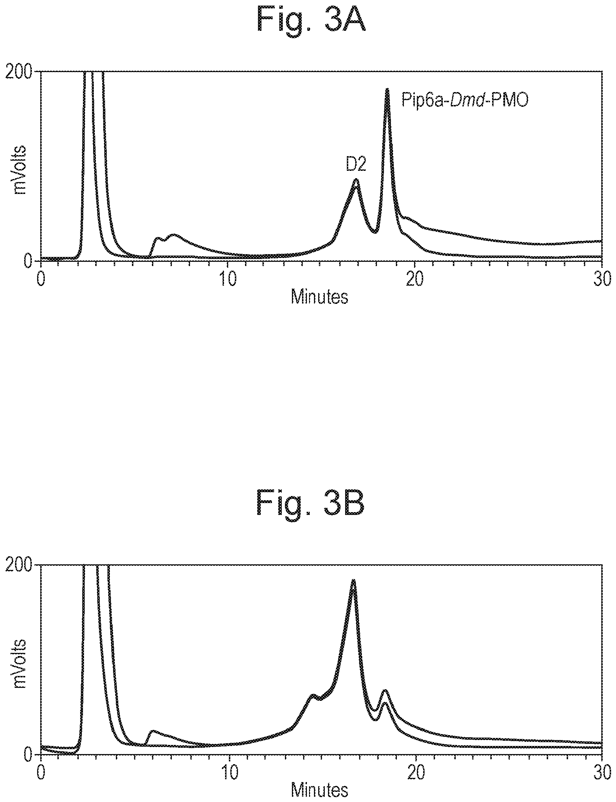

**Figure 3****.** Cation-exchange chromatogram in the purification of bi-specific conjugate D2 synthesised by alkyne-azide click chemistry. The copper-(I)-mediated click reaction was carried out using similar conditions but in **A** at room temperature for 6 h and in **B** at 40°C for 30 min.

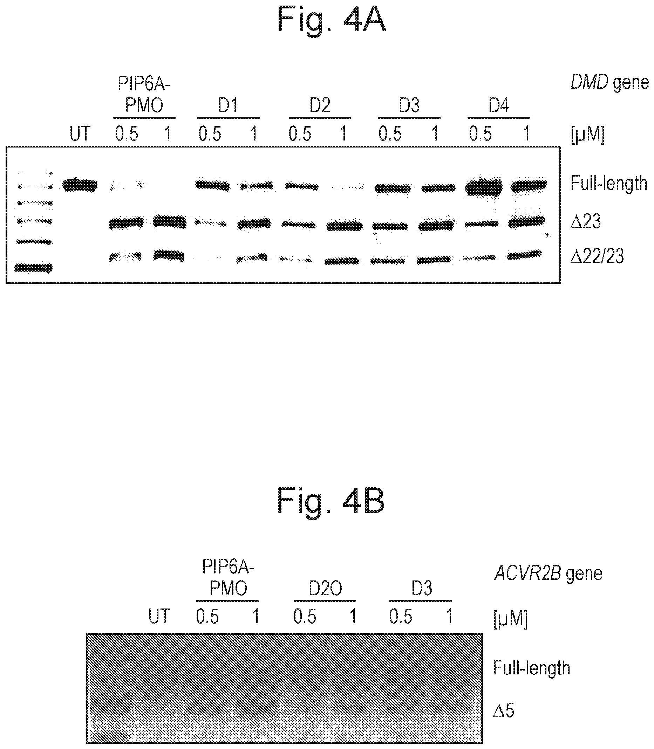

**Figure 4****.** RT-PCR analysis of (A) *Dmd* and (B) *Acvr2b* exon skipping activity of singular and bi-specific P-PMO conjugates in *H₂K mdx* cells treated for 4 hours with 0.5 µM or 1 µM concentrations of conjugates.

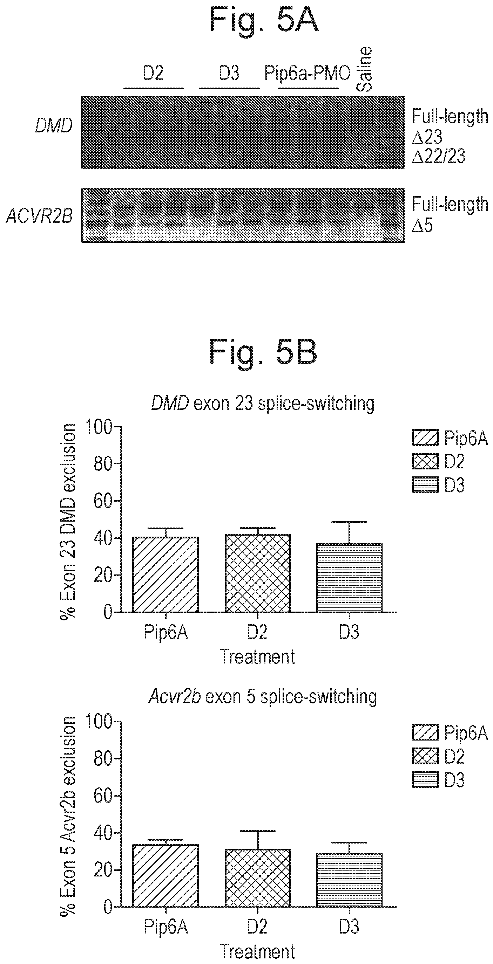

**Figure 5****.** Splice-switching activities of bi-specific (D2, D3) and a 1:1 molar cocktail of singly conjugated Pip6a-PMOs following intramuscular injection (N=3) into the *tibialis anterior* muscle of *mdx* mice. **A.** RT-PCR analysis *of Dmd* exon 23 and *Acvr2b* exon 5 removal from the mature transcripts. **B.** Quantitative PCR analysis *of Dmd* exon 23 and *Acvr2b* exon 5 skipping activity. Error bars = SEM

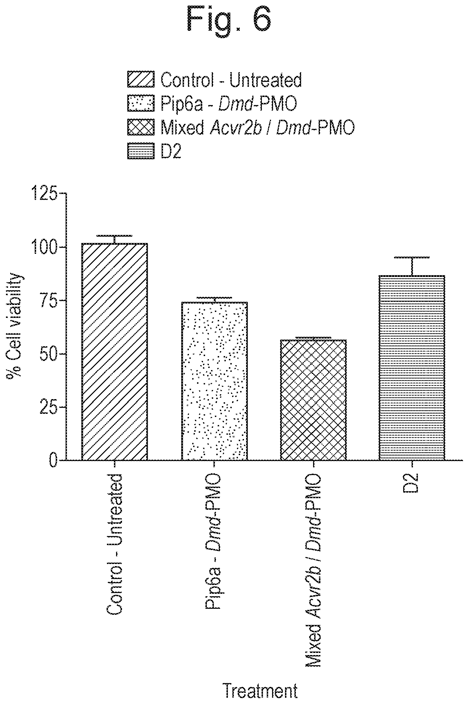

**Figure 6****.** Evaluation of the cell viabilities of Pip6a-PMO *(Dmd)* (20 µM), a mixture of Pip6a-PMO (*Dmd*) and Pip6a-PMO *(Acvr2b)* (10 µM each), and bi-specific P-PMO conjugate D2 (20 µM) in human hepatocytes (Huh7).

### Detailed Description of the Invention

It is to be understood that different applications of the disclosed products, uses and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "an oligonucleotide" includes two or more such oligonucleotides and the like.

### Cell penetrating peptide (CPP)

A "peptide" is used herein in its broadest sense to refer to a compound of two or more subunit amino acids, amino acid analogs, or other peptidomimetics. As used herein, the term "amino acid" refers to either natural and/or non-natural (unnatural or synthetic) amino acids, including both D or L optical isomers, and amino acid analogs and peptidomimetics.

Cell penetrating peptides (CPPs), sometime referred to as membrane translocating peptides, are a class of short peptides which are capable of targeting to and/or penetrating the membrane of a cell, permitting delivery to the interior of said cell. CPPs are typically used to facilitate the cellular uptake of various molecules (from nanosize particles to small chemical molecules and large fragments of DNA) which are attached as cargo to a CPP by covalent or non-covalent linkage. A CPP may thus be used for the *in vitro* and *in vivo* cellular delivery of a cargo molecule attached to the CPP. A number of defined classes of CPP are recognised in the art. These are described in more detail below and are encompassed in the definition of CPP used herein. However, it will be recognised that said definition also encompasses any peptide which exhibits the stated cell delivery properties.

A CPP, as with any peptide, may be synthesised using standard techniques known in the art, such as Fmoc solid phase chemistry, Boc solid phase chemistry or by solution phase peptide synthesis. Peptide synthesis methods using Fmoc are described in the Examples. Alternatively, a CPP, as with any peptide, may be purchased.

A CPP which may be included in a molecule of the invention preferably has the functional property of being able to target and/or penetrate into the interior of a mammalian cell. Said cell is preferably a human cell, preferably a muscle cell, heart cell, smooth muscle cell, liver cell, lung cell, brain cell, spinal cord cell, or any other neuron. This functional property of a CPP is not abolished by its inclusion in a molecule of the invention. In other words, when included in the molecule of the invention, the CPP preferably results in the targeting to and/or penetration into the interior of the cell of the entire molecule, including in particular the two cargo oligonucleotides. Preferably the entire molecule is delivered to the interior of the cell.

The functional properties of a CPP, or of any molecule of the invention comprising a CPP, may be assessed by any suitable method. Suitable methods are well-known. For example, delivery to a cell may be directly visualised by attachment of a suitable label, such as a fluorescent label, to the molecule to be tested. The labelled molecule is then incubated with cells in vitro, before the amount of label which has been internalised is quantified, e.g. with a fluorescence microscope. Alternatively, cell delivery may be assessed by a secondary indicator, such as the activity in a cultured cell of a cargo attached to the CPP or the molecule comprising the CPP. The level of cargo activity will indicate the level of cell delivery. For example, where an oligonucleotide cargo is designed to induce exon-skipping, the extent to which this has occurred can be assessed by any suitable method, such as RT-PCR analysis or assessing change in the levels of any protein products expressed or the function of the exon-skipped gene. Methods of this type are described in the Examples.

A preferred CPP to be included in a molecule of the invention is a PIP series CPP. PIP series CPPs are described in detail in WO2009/147368 and WO2013/030569. See in particular the general formulae provided in claims 1 to 15 of WO2013/030569 and claims 1 to 12 of WO2009/147368. Specific examples of PIP series CPPs are set out in Figures 18, 23 to 30 and 41 of WO2013/030569 and Figures 16 and 25 of WO2009/147368. See also Figures 1 to 10 and 11 to 12 of this specification. Each of these individual examples of a PIP series CPP may be used in the present invention. Pip6a is a particularly preferred example of a PIP series CPP and is particularly preferred for inclusion in a molecule of the invention. Pip6a consists of the amino acid sequence RXRRBRRXRYQFLIRXRBRXRB wherein X is 6-aminohexanoic acid (Ahx) and B is beta-Alanine (SEQ ID NO: 1). Another particularly preferred CPP for inclusion in a molecule of the invention is a peptide of formula (RXRRBR)₂XB, wherein X is 6-aminohexanoic acid (Ahx) and B is beta-Alanine (full sequence RXRRBRRXRRBRXB; SEQ ID NO: 2; also known as B peptide).

Other CPPs which may be included in a molecule of the invention are:
- Tat peptide (GRKKRRQRRRPPQ; SEQ ID NO: 3);
- Transportan (GWTLNSAGYLLGKINLKALAALAKKIL; SEQ ID NO: 4);
- Penetratin (RQIKIWFQNRRMKWKK; SEQ ID NO: 5);
- R6-Penetratin (RRRRRRRQIKIWFQNRRMKWKK; SEQ ID NO: 6);
- pVEC (LLIILRRRIRKQAHAHSK; SEQ ID NO: 7);
- MPG (GALFLGFLGAAGSTMGAWSQPKKKRKV; SEQ ID NO: 8)
- Pep1 (KETWWETWWTEWSQPKKKRKV; SEQ ID NO: 9)
- MAP (KLALKLALKALKAALKLA; SEQ ID NO: 10);
- R6W3 (RRWWRRWRR; SEQ ID NO: 11);
- A peptide of formula Rₙ, wherein 6<n<12;
- A peptide of formula (RXR)₄, wherein X is 4-aminobutyric acid (Abu), 6-aminohexanoic acid (Ahx), or 8-aminocaprylic acid (Acy), preferably Ahx;
- X9R wherein X is any cell or tissue-targeting sequence and 9R is nine arginine residues added C terminal to X; or
- RVG9R (YTIWMPENPRPGTPCDIFTNSRGKRASNGGGGRRRRRRRRRR; SEQ ID NO: 12).

Certain of the CPP classes described above are naturally occurring sequences. For example: Tat is residues 48-60 of the HIV Tat protein; Penetratin is the membrane localisation sequence (residues 43-58) of Drosophila protein Antennapedia; and pVEC is the internalisation sequence (residues 615-632) of Cadherin.

Other CPP classes are chimeric molecules. For example: MPG combines elements of HIV-gp41 and SV40 T-antigen; Pep-1 combines elements of HIV-reverse transcriptase and SV40 T-antigen.

Other classes of CPP are synthetic and may be designed de novo, or be based on the motifs present in the naturally occurring sequences. For example, R6-Penetratin and R6W3 are both based on Penetratin; The Rₙ, 6<n<12 peptides are based on Tat. RVG9R is based on a sequence derived from rabies virus glycoprotein (RVG) with 9 arginine residues added at the carboxy terminus.

It will be recognised that further CPPs suitable for inclusion in the molecules of the invention include any other naturally occurring cell-targeting or tissue-targeting sequence, or any naturally occurring membrane localisation or internalisation sequence. Similarly a CPP may be a chimeric molecule which combines elements of such sequences, or any related sequence based on the motifs present in the naturally occurring sequences, provided in every case that the resulting CPP possesses the required cell delivery properties. These sequences may be combined with other elements from other known CPPs. For example, as shown above a CPP may have the sequence X9R, wherein X is any cell-targeting or tissue-targeting sequence and 9R is 9 arginine residues added at the carboxy or amino terminus of X. Examples of X include the cell-targeting or tissue-targeting sequences of the other specific CPPs disclosed above.

### Linker moieties

A molecule of the invention includes at least two linker moieties, (L1) and (L2). Said linker moieties may be incorporated within the molecule of the invention using any suitable technique. Standard techniques for the incorporation of a linker within a molecule are known in the art. For example, where the linker moiety is an amino acid, the linker may typically be incorporated (directly to the CPP or via a spacer) using any standard peptide synthesis method. The linker may thus be incorporated during the synthesis of the CPP using any standard synthesis method, such as Fmoc solid phase chemistry, Boc solid phase chemistry or by solution phase peptide synthesis. Methods using Fmoc solid phase chemistry are described in the Examples.. In one embodiment of the invention, one of the linker moieties may be the C terminal amino acid of the CPP.

Each linker moiety is independently capable of attachment to a cargo molecule. Said attachment is typically through a covalent linkage although non-covalent interactions are also possible.

Any suitable covalent linkage may be used. Examples of suitable linkages include a disulphide bond, a thioether linkage, a thiol-maleimide linkage, an amide linkage, an oxime linkage, a morpholino linkage, or a click reaction.

A linker moiety as used herein may be any moiety which is capable of forming a covalent linkage with a cargo molecule. Preferably the moiety is one which is capable of forming a disulphide bond, a thioether linkage, a thiol-maleimide linkage, an amide linkage, an oxime linkage, or a morpholino linkage with a cargo molecule, or which is capable of performing a click reaction with a cargo molecule.

Examples of linker moieties capable of forming a disulphide bond include cysteine. Examples of linker moieties capable of forming a thioether linkage include cysteine. Examples of linker moieties capable of forming a thiol-maleimide linkage include cysteine. Examples of linker moieties capable of forming an oxime linkage include an aminooxy group. Examples of linker moieties capable of forming a thiazolidine linkage include cysteine. Examples of linker moieties capable of performing a click reaction include any amino acid (typically a non-natural amino acid) which includes a free alkyne moiety. bis-Homopropargylglycine and a contrained cyclo-octyne are preferred options.

Examples of linker moieties capable of forming a amide linkage include any amino acid. As used herein, the term "amino acid" refers to either natural and/or non-natural (unnatural or synthetic) amino acids, including both D or L optical isomers, and amino acid analogs and peptidomimetics. The 20 standard proteinogenic L-amino acids are shown in the following table. Not shown in the table are selenocysteine, pyrrolysine and N-formylmethionine which may also be considered proteinogenic.

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

Non-natural (unnatural or synthetic) amino acids are known to the person skilled in the art. Preferred non-natural amino acids include 6-aminohexanoic acid (Axh), 4-aminobutyric acid (Abu), 8-aminocaprylic acid (Acy), beta-Alanine, p-aminobenzoic acid and isonipecotic acid. Beta-Alanine is particularly preferred for inclusion in a molecule of the invention as a linker moiety.

Certain of the linker moieties and linkage types described above may require that the cargo molecule to which they are to be attached be functionalised to enable the linkage to form. Functionalisation typically means the modification of the oligonucleotide cargo molecule to include a species at either the 5' or 3' end which will react with the linker moiety to form the desired linkage. Examples of this type include the functionalisation of an olignucleotide with an azido group by coupling the free 3'-secondary amine group with an Fmoc-azido-amino acid. Under appropriate conditions the azido group may then participate in alkyne-azide click chemistry to form a linkage with a linker moiety comprising a free alkyne group. This type of linking is described in the Examples. Another example is the functionalisation of an oligonucleotide with a thiol activating group at the 3' end, for example by coupling to a 3-nitro-2-pyridinsulphenyl (Npys) group. Under appropriate conditions, the thiol-activating group may present a free sulfhydryl group which can form a disulphide bond with a linker moiety comprising a free thiol group. This type of linking is also described in the Examples.

Other of the linker moieties and linkage types described above require no specific modification of the oligonucleotide cargo in order for the desired linkage to form. That is, 5' or 3' terminal nucleotide of an oligonucleotide will react with the linker moiety to form the desired linkage. Examples of this latter type include the formation of an amide linkage between the secondary amine at the 3' end of an oligonucleotide (such as a morpholino oligonucleotide) and the free alpha carbon carboxyl group of an amino acid linker moiety at the C-terminus of a peptide. This type of linking is also described in the Examples.

### Spacer moieties

A molecule of the invention may include none, one or two spacer moieties, referred to as (S1) and (S2). If present, the spacer moieties (S1) and (S2) may each independently be a peptide of up to 5 amino acids in length, but may be 4, 3, 2 or 1 amino acid in length. If present, each of (S1) and (S2) is preferably independently 1 or 2 amino acids in length, most preferably 1 amino acid in length.

If present, each of (S1) and (S2) may each independently comprise or consist of an amino acid. As in the section relating to linker moieties, said amino acid may be any natural and/or non-natural (unnatural or synthetic) amino acid. Preferred amino acids for inclusion in a molecule of the invention in (S1) and (S2) include: alanine, glycine, arginine, 6-aminohexanoic acid (Axh), 4-aminobutyric acid (Abu), 8-aminocaprylic acid (Acy), beta-Alanine, p-aminobenzoic acid, and isonipecotic acid. 6-aminohexanoic acid (Axh) is particularly preferred.

The spacer moieties are typically included in a molecule of the invention as required to reduce or prevent an unwanted interaction between any other parts of the molecule. For example to reduce or prevent steric collision between the two oligonucleotide cargo molecules and also to provide more space for each oligonucleotide to interact with its target sequence by hybridisation whilst carrying out its function.

As with the linker moieties described above, standard techniques for the incorporation of a spacer within a molecule are known in the art. Since the spacer is one or more amino acids, it may typically be incorporated (directly to the CPP and/or linker) using any standard peptide synthesis method. The spacer may thus be incorporated during the synthesis of the CPP and/or linker using any standard synthesis method, such as Fmoc chemistry, Boc solid phase chemistry or by solution phase peptide synthesis. Methods using Fmoc chemistry are described in the Examples.

### Oligonucleotide cargo molecules and target sequences

Each of the two linker moieties present in a molecule of the invention, (L1) and (L2), is independently attached to a separate cargo molecule which comprises or consists of an oligonucleotide. The oligonucleotide may be functionalised as described above to facilitate or enable attachment to a linker moiety. Oligonucleotides may be synthesised using standard techniques known in the art. Alternatively, oligonucleotides may be purchased.

An oligonucleotide is a short polymer comprising two or more nucleotides. Typically an oligonucleotide is no more 20, 25, 30, 35, 40, 45 or 50 nucleotides in length. Preferably an oligonucleotide is 20 to 25 nucleotides in length. The nucleotides can be naturally occurring or artificial. A nucleotide typically contains a nucleobase, a sugar and at least one linking group, such as a phosphate, phosphoramidate, methylphosphonate or phosphorothioate group. The nucleobase is typically heterocyclic. Nucleobases include, but are not limited to, purines and pyrimidines and more specifically adenine (A), guanine (G), thymine (T), uracil (U) and cytosine (C). The sugar is typically a pentose sugar. Nucleotide sugars include, but are not limited to, ribose and deoxyribose. The nucleotide is typically a ribonucleotide or deoxyribonucleotide. Phosphates may be attached on the 5' or 3' side of a nucleotide.

Nucleotides include, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (UDP), uridine triphosphate (UTP), cytidine monophosphate (CMP), cytidine diphosphate (CDP), cytidine triphosphate (CTP), 5-methylcytidine monophosphate, 5-methylcytidine diphosphate, 5-methylcytidine triphosphate, 5-hydroxymethylcytidine monophosphate, 5-hydroxymethylcytidine diphosphate, 5-hydroxymethylcytidine triphosphate, cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP), 5-methyl-2'-deoxycytidine monophosphate, 5-methyl-2'-deoxycytidine diphosphate, 5-methyl-2'-deoxycytidine triphosphate, 5-hydroxymethyl-2'-deoxycytidine monophosphate, 5-hydroxymethyl-2'-deoxycytidine diphosphate and 5-hydroxymethyl-2'-deoxycytidine triphosphate. The nucleotides are preferably selected from AMP, TMP, GMP, UMP, dAMP, dTMP, dGMP or dCMP.

The nucleotides may contain additional modifications. In particular, suitable modified nucleotides include, but are not limited to, 2'amino pyrimidines (such as 2'-amino cytidine and 2'-amino uridine), 2'-hyrdroxyl purines (such as, 2'-fluoro pyrimidines (such as 2'-fluorocytidine and 2'fluoro uridine), hydroxyl pyrimidines (such as 5'-α-P-borano uridine), 2'-O-methyl nucleotides (such as 2'-O-methyl adenosine, 2'-O-methyl guanosine, 2'-O-methyl cytidine and 2'-O-methyl uridine), 2'-O-methoxy-ethyl nucleotides, 4'-thio pyrimidines (such as 4'-thio uridine and 4'-thio cytidine) and nucleotides having modifications of the nucleobase (such as 5-pentynyl-2'-deoxy uridine, 5-(3-aminopropyl)-uridine and 1,6-diaminohexyl-N-5-carbamoylmethyl uridine).

One or more nucleotides in the oligonucleotide can be oxidized or methylated. One or more nucleotides in the oligonucleotide may be damaged. For instance, the oligonucleotide may comprise a pyrimidine dimer. Such dimers are typically associated with damage by ultraviolet light.

The nucleotides in the oligonucleotide may be attached to each other in any manner. The nucleotides may be linked by phosphate, 2'O-methyl, 2' methoxy-ethyl, phosphoramidate, methylphosphonate or phosphorothioate linkages. The nucleotides are typically attached by their sugar and phosphate groups as in nucleic acids. The nucleotides may be connected via their nucleobases as in pyrimidine dimers.

The oligonucleotide can be a nucleic acid, such as deoxyribonucleic acid (DNA) or a ribonucleic acid (RNA). The oligonucleotide may be any synthetic nucleic acid known in the art, such as peptide nucleic acid (PNA), glycerol nucleic acid (GNA), threose nucleic acid (TNA), locked nucleic acid (LNA), morpholino nucleic acid (such as phosphorodiamidate morpholino oligonucleotide (PMO)) or other synthetic polymers with nucleotide side chains. The oligonucleotide may be single stranded or double stranded.

Each oligonucleotide included in a molecule of the invention specifically hybridises to a target sequence. The length of the target sequence typically corresponds to the length of the oligonucleotide. For instance, a 20 or 25 nucleotide oligonucleotide typically specifically hybridises to a 20 or 25 nucleotide target sequence. The target sequence may therefore be any of the lengths discussed above with reference to the length of the oligonucleotide. The target sequence is typically consecutive nucleotides within a target polynucleotide.

An oligonucleotide "*specifically hybridises*" to a target sequence when it hybridises with preferential or high affinity to the target sequence but does not substantially hybridise, does not hybridise or hybridises with only low affinity to other sequences.

An oligonucleotide "*specifically hybridises*" if it hybridises to the target sequence with a melting temperature (Tₘ) that is at least 2 °C, such as at least 3 °C, at least 4 °C, at least 5 °C, at least 6 °C, at least 7 °C, at least 8 °C, at least 9 °C or at least 10 °C, greater than its Tₘ for other sequences. More preferably, the oligonucleotide hybridises to the target sequence with a Tₘ that is at least 2 °C, such as at least 3 °C, at least 4 °C, at least 5 °C, at least 6 °C, at least 7 °C, at least 8 °C, at least 9 °C, at least 10 °C, at least 20 °C, at least 30 °C or at least 40 °C, greater than its Tₘ for other nucleic acids. Preferably, the portion hybridises to the target sequence with a Tₘ that is at least 2 °C, such as at least 3 °C, at least 4 °C, at least 5 °C, at least 6 °C, at least 7 °C, at least 8 °C, at least 9 °C, at least 10 °C, at least 20 °C, at least 30 °C or at least 40 °C, greater than its Tₘ for a sequence which differs from the target sequence by one or more nucleotides, such as by 1, 2, 3, 4 or 5 or more nucleotides. The portion typically hybridises to the target sequence with a Tₘ of at least 90 °C, such as at least 92 °C or at least 95 °C. Tₘ can be measured experimentally using known techniques, including the use of DNA microarrays, or can be calculated using publicly available Tₘ calculators, such as those available over the internet.

Conditions that permit the hybridisation are well-known in the art (for example, Sambrook et al., 2001, Molecular Cloning: a laboratory manual, 3rd edition, Cold Spring Harbour Laboratory Press; and Current Protocols in Molecular Biology, Chapter 2, Ausubel et al., Eds., Greene Publishing and Wiley-lnterscience, New York (1995)). Hybridisation can be carried out under low stringency conditions, for example in the presence of a buffered solution of 30 to 35% formamide, 1 M NaCl and 1 % SDS (sodium dodecyl sulfate) at 37 °C followed by a 20 wash in from IX (0.1650 M Na⁺) to 2X (0.33 M Na⁺) SSC (standard sodium citrate) at 50 °C. Hybridisation can be carried out under moderate stringency conditions, for example in the presence of a buffer solution of 40 to 45% formamide, 1 M NaCl, and 1 % SDS at 37 °C, followed by a wash in from 0.5X (0.0825 M Na⁺) to IX (0.1650 M Na⁺) SSC at 55 °C. Hybridisation can be carried out under high stringency conditions, for example in the presence of a buffered solution of 50% formamide, 1 M NaCl, 1% SDS at 37 °C, followed by a wash in 0.1X (0.0165 M Na⁺) SSC at 60 °C.

The oligonucleotide may comprise a sequence which is substantially complementary to the target sequence. Typically, the oligonucleotides are 100% complementary. However, lower levels of complementarity may also be acceptable, such as 95%, 90%, 85% and even 80%. Complementarity below 100% is acceptable as long as the oligonucleotides specifically hybridise to the target sequence. An oligonucleotide may therefore have 1, 2, 3, 4, 5, 6, 7, 8, 9, 10 or more mismatches across a region of 5, 10, 15, 20, 21, 22, 30, 40 or 50 nucleotides. Thus, two oligonucleotides which each comprise a different sequence may nonetheless specifically hybridise to the same target sequence due to differing levels of complementarity with the target sequence, or due to specifically hybridising to target sequences which overlap with each other. The extent of the overlap may be at least 5, 10, 15, 16, 17, 18, 19, 20, 21, 22, 23, 24, 25, 30, 35, 40, 45 or more nucleotides.

An oligonucleotide included in a molecule of the invention interferes with a function of a cell when delivered to and/or into said cell. More specifically, the oligonucleotide is preferably an antisense or a RNAi molecule. Both antisense and RNAi molecules interfere with the function of naturally occurring RNA molecules in cells. Antisense oligonucleotides interfere with RNA by binding to (hybridising with) a section of the RNA. RNAi involves the use of double-stranded RNA, such as small interfering RNA (siRNA) or small hairpin RNA (shRNA), which can bind to the RNA and inhibit function. Thus, an oligonucleotide included in a molecule of the invention typically interferes with the function of an RNA molecule in a cell, and the sequence targeted by said oligonucleotide is typically consecutive nucleotides of the polynucleotide sequence of said RNA molecule.

The oligonucleotide is therefore designed to be complementary to the RNA (although the oligonucleotide does not have to be 100% complementary to the target sequence as discussed above). By way of illustration, where the RNA molecule is messenger RNA (mRNA, discussed further below), the oligonucleotide may be considered to be equivalent to a section of cDNA, but need not be 100% identical to the cDNA sequence.

The RNA molecule to which the oligonucleotide is complementary may have been transcribed from a coding element of the genome of a cell (i.e. from a gene) and hence may be messenger RNA (mRNA) or pre-messenger RNA (pre-mRNA). In either case, the function interfered with is expression of the said gene. A particular example of interference with gene expression in this way is exon-skipping. In exon-skipping, the target sequence comprises a mutation in an exon of a gene. The oligonucleotide hybridises to the exon in the pre-mRNA molecule, resulting in that exon being "skipped" (missed out) during splicing. The result is a truncated mature mRNA which lacks the mutated exon, but can be translated into a functional protein.

Alternatively, the RNA molecule to which the oligonucleotide is complementary may have been transcribed from a non-coding element of the genome. RNA molecules transcribed from non-coding elements of the genome include ribosomal RNA, transfer RNA, Piwi-interacting RNA, microRNA, and long non coding RNA (lncRNA). Natural antisense transcripts (NAT) are a sub-category of IncRNA. Both microRNA and NAT are examples of naturally occurring molecules which function to silence or reduce expression by interfering with mRNA. Thus, an oligonucleotide which interferes with the function of a microRNA or NAT may result in increased expression of a gene which would otherwise be suppressed by the naturally occurring microRNA or NAT.

The target sequence may be present in any of the types of RNA molecule described above. The target sequence is preferably associated with a disease or condition. Said disease or condition may be a muscle-related disease or condition, a heart-related disease or condition, a neurological disease or condition, or cancer.

Muscle-related diseases and conditions include: Becker muscular dystrophy, Bethlem myopathy, Central core disease, Charcot-Marie-Tooth disease (CMT), Congenital muscular dystrophy (CMD), Congenital myasthenic syndromes, Congenital myotonic dystrophy, Duchenne muscular dystrophy, Emery-Dreifuss muscular dystrophy, Facioscapulohumeral muscular dystrophy (FSH), Fibre-type disproportion, Fibrodysplasia ossificans progressiva (FOP), Inclusion body myositis (IBM), Juvenile dermatomyositis, Limb girdle muscular dystrophies (LGMD), Limb girdle muscular dystrophy 1B (LGMD 1B), Limb girdle muscular dystrophy 1C (LGMD 1C), Limb girdle muscular dystrophy 2A (LGMD 2A), Limb girdle muscular dystrophy 2B (LGMD 2B), Limb girdle muscular dystrophy 2I (LGMD 21), Manifesting carriers of Duchenne muscular dystrophy, McArdle disease, Merosin-deficient congenital muscular dystrophy: MDC1A, Metabolic disorders, Minicore (multicore) myopathy, Mitochondrial myopathies, Myasthenia gravis, Myopathy, Myotonias, Myotonic dystrophy, Myotubular (centronuclear) myopathy, Nemaline myopathy, Oculopharyngeal muscular dystrophy (OPMD), Periodic paralyses, Polymyositis, dermatomyositis and sarcoid myopathy, Rigid spine syndrome, Sarcoglycanopathies: LGMD2C, LGMD2D, LGMD2E and LGMD2F, Spinal muscular atrophy (SMA), and Ullrich congenital muscular dystrophy.

The muscle-related condition is preferably any form of muscular dystrophy, particularly preferably DMD.

Neurological diseases or conditions include multiple sclerosis and related neuroinflammatory conditions (such as Acute disseminated encephalomyelitis (ADEM), Optic Neuritis (ON), Transverse Myelitis, and Neuromyelitis Optica (NMO)), and neuroinflammation associated with neurodegeneration.

The target sequence may be a sequence in a RNA molecule associated with any one of the diseases or conditions described above. The target sequence may preferably be in a mRNA or pre-mRNA transcribed from a murine or human gene associated with said disease or condition, but may be in a RNA transcribed from a non-coding element of the murine or human genome associated with said disease or condition. By "associated with a disease or condition" it is typically meant that a target sequence includes a mutation relative to the wild type form of a sequence. The wild type form is the corresponding sequence in a healthy individual.

Target sequences may include those which comprise a mutation in an exon of a gene that disrupts the open reading frame of the gene. Such mutations typically result in a C-terminally truncated, often non-functional form of the encoded protein. Oligonucleotides which specifically hybridise to target sequences which comprise a mutation in an exon preferably induce skipping of the mutated exon during pre-mRNA splicing, resulting in an internally deleted, but functional protein. Such oligonucleotides are preferred for inclusion in a molecule of the invention.

Examples of target sequences which comprise a mutation in an exon include the mutated sequences which are associated with muscular dystrophy. Accordingly target sequences of particular interest include those comprising mutations in the pre-mRNA transcribed from the murine or human dystrophin gene. Oligonucleotides which specifically hybridise to such target sequences (and hence molecules of the invention comprising such oligonucleotides) may be used to treat muscular dystrophy and hence are particularly preferred. Said target sequences may be within any exon of the human dystrophin gene, but are preferably within exons 44 to 55 inclusive. Examples of oligonucleotides comprising sequences which specifically hybridise to target sequences in the pre-mRNA transcribed from the human dystrophin gene include those disclosed in US Patent No. 8,637,483 as SEQ ID NOs: 1 to 62. Said oligonucleotide sequences are also shown in Table 1A of US Patent No. 8,637,483. The oligonucleotide sequences of SEQ ID NOs: 1 to 62 and Table 1A of US Patent No. 8,637,483 may be used in the present invention.

Further examples of oligonucleotides comprising sequences which specifically hybridise to target sequences in the pre-mRNA transcribed from the human dystrophin gene include those disclosed in International Patent Publication No. WO2006/000057 (International Application No. PCT/AU2005/000943) as SEQ ID NOs: 1 to 202 or as shown in Table 1 of International Patent Publication No. WO2006/000057. The oligonucleotide sequences of SEQ ID NOs: 1 to 202 and Table 1 of WO2006/000057 may be used in the present invention.

Other examples of oligonucleotides comprising sequences which specifically hybridise to target sequences in the pre-mRNA transcribed from the human dystrophin gene include an oligonucleotide which comprises or consists of:
- the sequence GGCCAAACCTCGGCTTACCT (SEQ ID NO: 13) or a sequence having at least 90% sequence identity to said sequence; or
- the sequence GGCCAAACCTCGGCTTACCTGAAAT (SEQ ID NO: 14) or a sequence having at least 90% sequence identity to said sequence; or

Target sequences may alternatively include those which comprise a mutation in an intron of a human gene that disrupts correct splicing of the pre-mRNA transcribed from the gene. Such mutations may result in a protein with an internal deletion (lacking an exon), which may be non-functional. Oligonucleotides which specifically hybridise to target sequences which comprise a mutation in an intron preferably suppress the exclusion of a subsequent exon during pre-mRNA splicing, resulting in a functional protein which includes the exon. Such oligonucleotides are preferred for inclusion in a molecule of the invention.

Examples of target sequences which comprise a mutation in an intron include mutated sequences which are associated with spinal muscular atrophy (SMA). Oligonucleotides which specifically hybridise to such target sequences (and hence molecules of the invention comprising such oligonucleotides) may be used to treat SMA and hence are particularly preferred. Linkage mapping identified the Survival of Motor Neuron (SMN) gene as the genetic locus of SMA. In humans, two nearly identical SMN genes (SMN1 and SMN2) exist on chromosome 5q13. Deletions or mutations within SMN1 but not the SMN2 gene cause all forms of proximal SMA. SMN1 encodes a ubiquitously expressed 38 kDa SMN protein that is necessary for snRNP assembly, an essential process for cell survival. A nearly identical copy of the gene, SMN2, fails to compensate for the loss of SMN1 because exon 7 is skipped, producing an unstable truncated protein, SMNΔ7. SMN1 and SMN2 differ by a critical C to T substitution at position 6 of exon 7 (C6U in transcript of SMN2). C6U does not change the coding sequence, but is sufficient to cause exon 7 skipping in SMN2. Any therapy which can improve the levels of exon 7 inclusion in SMN2 is likely to be highly beneficial. Accordingly, target sequences within SMN2 pre-mRNA are of interest, including in particular an intronic inhibitory sequence element, named ISS-N1 (for "intronic splicing silencer"), in SMN2 pre-mRNA. The ISS-N1 sequence is specifically disclosed in US Patent No 7,838,657. A preferred form of the ISS-N1 sequence is disclosed as SEQ ID NO: 3 of US Patent No 7,838,657. That sequence is 5'-CCAGCAUUAUGAAAG-3' and is also included herein as SEQ ID NO: 18.
Oligonucleotides which specifically hybridise to the ISS-N1 sequence, resulting in enhanced inclusion of exon 7 during splicing of SMN2 pre-mRNA, are preferred for inclusion in molecules of the invention. Oligonucleotide comprising sequences which specifically hybridise to the ISS-N1 sequence are also disclosed in US Patent No 7,838,657, see in particular SEQ ID NO: 4 of that document. That sequence is 5'-CUUUCAUAAUGCUGG-3' and is also included herein as SEQ ID NO: 17. A molecule of the invention may include an oligonucleotide which comprises or consists of the sequence 5'-CUUUCAUAAUGCUGG-3' (SEQ ID NO: 17) or a sequence having at least 90% sequence identity to said sequence.

Other target sequences of interest include those in mRNA or pre-mRNA transcribed from the human Activin receptor type-2B gene (ACVR2B), which is also associated with muscular dystrophy. A specific example of an oligonucleotide comprising sequence which specifically hybridises to a target sequences in the ACVR2B gene is an oligonucleotide which comprises or consists of the sequence GCCTCGTTTCTCGGCAGCAATGAAC (SEQ ID NO: 15) or a sequence having at least 90% sequence identity to said sequence.

### Treatment and Administration

A molecule of the invention may be used in a method of treating a disease or condition as defined above. Said method comprises administering the molecule of the invention to a patient. A molecule of the invention may be administered to the patient in any appropriate way. In the invention, the molecule may be administered in a variety of dosage forms. Thus, it can be administered orally, for example as tablets, troches, lozenges, aqueous or oily suspensions, dispersible powders or granules. It may also be administered by enteral or parenteral routes such as via buccal, anal, pulmonary, intravenous, intra-arterial, intramuscular, intraperitoneal, intraarticular, topical or other appropriate administration routes. The molecule may be administered directly into the cancer to be treated. A physician will be able to determine the required route of administration for each particular patient.

The formulation of a molecule will depend upon factors such as the nature of the exact inhibitor, etc. A molecule may be formulated for simultaneous, separate or sequential use with other inhibitors defined herein or with other cancer treatments as discussed in more detail below.

A molecule is typically formulated for administration as a pharmaceutical composition, which may include a pharmaceutically acceptable diluent, adjuvant or carrier. The pharmaceutical carrier or diluent may be, for example, an isotonic solution. For example, solid oral forms may contain, together with the active substance, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active substance, a pharmaceutically acceptable carrier, e.g. sterile water, olive oil, ethyl oleate, glycols, e.g. propylene glycol, and if desired, a suitable amount of lidocaine hydrochloride.

Solutions for intravenous administration or infusion may contain as carrier, for example, sterile water or preferably they may be in the form of sterile, aqueous, isotonic saline solutions.

For suppositories, traditional binders and carriers may include, for example, polyalkylene glycols or triglycerides; such suppositories may be formed from mixtures containing the active ingredient in the range of 0.5% to 10%, preferably 1% to 2%.

Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the pharmaceutical composition is lyophilised, the lyophilised material may be reconstituted prior to administration, e.g. a suspension. Reconstitution is preferably effected in buffer.

Capsules, tablets and pills for oral administration to an individual may be provided with an enteric coating comprising, for example, Eudragit "S", Eudragit "L", cellulose acetate, cellulose acetate phthalate or hydroxypropylmethyl cellulose.

The molecule of the invention may be delivered in combination with cationic lipids, polymers or targeting systems. They may be delivered by any available technique. For example, the molecule may be introduced by needle injection, preferably intradermally, subcutaneously or intramuscularly. Alternatively, the molecule may be delivered directly across the skin using a delivery device such as particle-mediated gene delivery. The molecule may be administered topically to the skin, or to mucosal surfaces for example by intranasal, oral, or intrarectal administration.

A therapeutically effective amount of the molecule is typically administered to the patient. A therapeutically effective amount of is an amount effective to ameliorate one or more symptoms of the disease or condition to be treated. A therapeutically effective amount of the molecule is preferably an amount effective to abolish one or more of, or preferably all of, the symptoms of the disease or condition.

The dose may be determined according to various parameters, especially according to the substance used; the age, weight and condition of the patient to be treated; the route of administration; and the required regimen. Again, a physician will be able to determine the required route of administration and dosage for any particular patient. A typical daily dose is from about 0.1 to 50 mg per kg of body weight, according to the activity of the specific inhibitor, the age, weight and conditions of the subject to be treated and the frequency and route of administration. The dose may be provided as a single dose or may be provided as multiple doses, for example taken at regular intervals, for example 2, 3 or 4 doses administered hourly. Preferably, dosage levels of inhibitors are from 5 mg to 2 g.

Typically the molecule may be administered in the range of 1 pg to 1 mg, preferably to 1 pg to 10 µg nucleic acid for particle mediated delivery and 10 µg to 1 mg for other routes.

### Examples

### Example 1

Preliminary data (not shown) was obtained where two copies of an identical exon-skipping morpholino oligonucleotide (targeting exon 23 of murine dystrophin) are coupled together via a disulfide bond and linked to a CPP. The Pip5e CPP was used. This has the sequence RXRRBRRXRILFQYRXRBRXRB (SEQ ID NO: 16); wherein X is 6-aminohexanoic acid (Ahx) and B is beta-Alanine. The first oligo was functionalised with a free S-S-R group at the 3', in order to generate a free thiol group following a reduction step, before coupling of the 5' end of the morpholino oligo prefurnished with an amino linker to the C-terminus of the CPP by standard amide linkage. The second oligo, functionalised at the 3' end with an Npys group, was then added under conditions suitable for disulphide bond formation between the 3' ends of each oligo.

Exon skipping activity in mdx muscle cells for the bi-coupled molecule was found not to have doubled but was improved relative to that for a the same CPP coupled to a single copy of the oligonucleotide. Thus there is proof of principle that two identical oligonucleotides (or two oligonucleotides specific for the same or closely related targets) can be coupled to a CPP such that both retain at least some activity in cells.

It was hypothesised that the presence of the disulfide bond linking the oligonucleotides could in principle generate a free thiol group inside cells. Evidence was found to suggest that this may have been deleterious to exon skipping activity. Subsequent experiments therefore sought to develop alternative chemistries for attachment of two oligonucleotide cargo molecules to a CPP, such that a greater level of activity is retained for each cargo.

### Example 2

Starting with the PIP6a CPP (SEQ ID NO: 1) different chemistries for the attachment of two oligonucleotides were tested. Three different conjugation chemistries, including amide, disulfide and triazole bonds were utilised to allow orthogonal conjugation. The oligonucleotides used were an exon-skipping PMO targeting exon 23 of the murine dystrophin *(Dmd)* gene to correct the *mdx* genotype, and an exon-skipping PMO targeting exon 5 of the *Acvr2b* gene so as to produce an internally deleted protein that lacks the crucial trans-membrane domains. Thus, the two cargo molecules are not identical and do not target the same or closely-related sequences. However, the same attachment techniques apply equally to identical cargo molecules, or cargo molecules specific for the same or closely-related target sequences.

Several different bi-specific conjugate designs were investigated whereby the two PMOs were joined either at one end of the Pip6a peptide or with one PMO at either N- or C-termini. The activities of these conjugate constructs were assessed in mouse *mdx* cells and the most active bi-specific conjugates, which had both PMOs attached at the C-terminus of the CPP, were shown to have closely comparable *Dmd* exon skipping activity to the single Pip6a-PMO targeting *Dmd.* These conjugates were also assessed in the same cells for targeting of *Acvr2b* and both conjugates demonstrated only very slightly reduced exon skipping activity compared to Pip6a-PMO targeting *Acvr2b.* Importantly, the cell viability using a bi-specific compound was significantly better than for a mixture of the two individual Pip6a-PMOs. The potential of this approach was further assessed in an *in vivo* environment through intramuscular administration. It was demonstrated that there were no significant differences in exon skipping activities for both *Dmd* and *Acvr2b* targets between bi-specific conjugates and a cocktail of the individual P-PMO equivalents.

### MATERIAL AND METHODS

### Materials

Fmoc-protected amino acids, coupling reagents (HBTU and PyBOP) and the Fmoc-β-Ala-OH preloaded Wang resin (0.19 mmol g⁻¹) were obtained from Merck (Hohenbrunn, Germany). Fmoc-azido-L-lysine-OH was from IRIS Biotech GMBH (Deutschland, Germany). Fmoc-L-bis-homopropargylglycine-OH (Bpg) was purchased from Chiralix (Nijmegen, The Netherlands). Chicken Embryo Extract and horse serum for cell culture were obtained from Sera Laboratories International Ltd (West Sussex, UK). γ-Interferon was obtained from Roche Applied Science (Penzberg, Germany). All other reagents were obtained from Sigma-Aldrich (St. Louis, MO, USA) unless otherwise stated. MALDI-TOF mass spectrometry (Table 1) was carried out using a Voyager DE Pro BioSpectrometry workstation. A stock solution of 10 mg mL⁻¹ of α-cyano-4-hydroxycinnamic acid or sinapinic acid in 60% acetonitrile in water was used as matrix. The measurements have an accuracy level of ± 0.1 %.

### Peptide Synthesis

Peptides were synthesized by standard Fmoc chemistry (34) using a CEM Liberty™ microwave peptide synthesizer (Buckingham, UK). Peptides were assembled on Fmoc-β-Ala-OH preloaded Wang resin on a 0.1 mmol scale with excess of Fmoc-protected amino acids, PyBOP and DIPEA (5:5:10). The Nα-Fmoc protecting groups were removed by treating the resin with piperidine in DMF (20% v/v) at 75 °C twice, once for 30 sec and then for 3 min. The coupling reactions were carried out at 75 °C for 5 min. In order to prevent racemisation, Fmoc-cysteine (Trt)-OH was coupled at 50 °C for 10 min at 60 watt microwave power. All amino acids were single coupled except for the arginines, which were double coupled. The Fmoc-L-bis-homopropargylglycine-OH was coupled manually using a 2 fold excess and the coupling success was checked using a TNBS test (35). After completion of peptide assembly, the resin bound peptide was cleaved off by treating the resin with a cocktail of TFA:DoDt:H₂O:TIPS (94:2.5:2.5:1, v/v) for 2 h. The peptide was precipitated by addition of ice-cold diethyl ether and washed three times. The crude peptides were analyzed and purified to more than 90% by reversed-phase HPLC (RP-HPLC). The peptide mass characterisation was carried out using a MALDI-TOF mass spectrometry (ABI Voyager DE Pro) and an α-cyano-4-hydroxycinnamic acid matrix made up in 70% acetonitrile containing 0.05 % TFA.

### Functionalisation of PMO

The PMO sequence for exon-23 skipping of *Dmd* pre-mRNA (5'-GGCCAAACCTCGGCTTACCTGAAAT) was either unmodified (standard morpholino with a secondary amine at the 3' end) or functionalized with a disulfide at its 3'-end. The PMO targeting exon-5 of *Acvr2b* was unmodified (5'-GCCTCGTTTCTCGGCAGCAATGAAC-3'). All PMOs were purchased from Gene Tools LLC (Philomath, USA). 3'-unmodified PMO was functionalized with an azido group by coupling the free 3'-secondary amine group with Fmoc-azido-L-Lysine-OH (Figure 1 A). The coupling was carried out by activating the carboxyl group of the amino acid derivative using HBTU (2.5 eq) and HOAt (2 eq.) in NMP in the presence of 2.5 eq. of DIEA before addition of the PMO dissolved in dimethylsulfoxide (DMSO). The Fmoc-azido-L-Lysine-PMO conjugate was then purified using RP-HPLC followed by Fmoc deprotection and purification. In the case of PMO with a disulfide bond at its 3' end (Figure 1B), the disulfide bond was reduced to give a free sulfhydryl group using a 10 fold excess of tris(2-carboxyethyl)phosphine hydrochloride (TCEP.HCl) in water for 1 h followed by filtration to remove the excess TCEP. The PMO with a free sulfhydryl group was then activated using a 2.5-fold molar excess of 2,2'-dithiobis(5-nitropyridine) (DTNP) in DMSO: acetonitrile (0.1% TFA): H₂O (0.1% TFA) with (1:1:3) ratios (36). The reaction mixture was stirred at room temperature for 2 h and the NPys-activated PMO was purified by RP-HPLC.

### Peptide-PMO conjugations

Conjugations of peptides to PMOs (Figure 2) were carried out in solution using a 2.5-fold excess of peptide using similar conditions to the coupling of Fmoc-azido-L-Lysine-OH to PMO. The conjugation of the second PMO was carried out using either copper (I) mediated alkyne-azide click chemistry between the alkyne-functionalised P-PMO (Figure 2A) and the azide-functionalised second PMO or by forming a disulfide bond between the 3' NPys-activated second PMO and a free cysteine thiol of the P-PMO (Figure 2B). The alkyne-azide click reaction between azide-functionalised PMO and alkyne functionalised P-PMO was carried out by dissolving the P-PMO in water followed by addition of azido-functionalised PMO (1.2 eq.). Sodium ascorbate (10 eq. as a 20 mM solution) was added and the reaction mixture was vortexed thoroughly followed by addition of copper (II)-TBTA (12 eq. as a 20 mM solution) (37). The click reaction was carried out at room temperature as well as at 40 °C. The conjugation of 3' NPys-activated PMO to P-PMO was carried out by first dissolving the Npys-activated PMO in ammonium bicarbonate solution (pH 8) followed by addition of the P-PMO dissolved in water. The reaction mixture was stirred at room temperature for 1 h.
The single and dual P-PMO conjugates were purified on a high-resolution (HR)-16 cation-exchange column (GE Healthcare, USA) using 25 mM sodium phosphate buffer (pH 7.2) containing 25% acetonitrile. The conjugates were eluted using a 1M NaCl solution in the same buffer at a flow rate of 6 ml min⁻¹. The excess salts were removed by centrifugation using an Amicon® Ultra-15 3K centrifugal filter device. The conjugates were characterized using MALDI-TOF MS as mentioned above. They were dissolved in sterile water and filtered through a 0.22 µm cellulose acetate membrane (Costar) before use.

### Intramuscular administration

Experiments were carried out in the Biomedical Sciences Unit, University of Oxford according to procedures authorized by the UK Home Office. Experiments were carried out in *mdx* mice (C57BL/10ScSn-Dmd^{mdx}/J) (38). Intramuscular (IM) injections (n=3 per treatment) were carried out on 24-week old *mdx* mice under general anaesthesia. 0.5 nmoles Peptide-PMO in 30 µl 0.9% saline volume was injected into *tibialis anterior* (TA) muscle. Two weeks post-administration animals were sacrificed by rising CO₂ inhalation and tissues snap-frozen in a dry ice cooled isopentane bath and stored at -80 °C.

### RT-PCR and qPCR analysis

RNA was extracted from either H2K*mdx* cell pellets or from TA tissue sections by mechanical disruption; and subsequently processed using Trizol according to manufacturer's instructions (Life Technologies). RT-PCR analysis of exon skipping levels was carried out with 400 ng of total RNA used as a template in a 50 µl RT-PCR using the GeneAmp RNA PCR kit (Applied Biosystems, Warrington UK). RT-PCR amplification of the dystrophin *Dmd* transcript was carried out under the following conditions: 95 °C for 20 seconds, 58 °C for 60 seconds and 72°C for 120 seconds for 30 cycles using the following primers: DysEx20Fo (5-CAGAATTCTGCCAATTGCTGAG) and DysEx26Ro (5'-TTCTTCAGCTTGTGTCATCC). 2 µl of this reaction was used as a template for nested amplification using Amplitaq Gold (Applied Biosystems, Warrington UK) under the following conditions: 95 °C for 20 seconds, 60 °C for 4 seconds, and 72°C for 120 seconds for 22 cycles using the following primers: DysEx20Fi (5'-CCCAGTCTACCACCCTATCAGAGC) and DysEx26Ri (5'-CCTGCCTTTAAGGCTTCCTT). *Acvr2b* RT-PCR amplification was carried out under the following conditions: 95 °C for 20 seconds, 60 °C for 45 seconds, and 72°C for 60 seconds for 35 cycles using the following primers: Acvr2bEx4F (5'-CTGCGTTTGGAAAGCTCAGCTCAT) and Acvr2bEx9R (5'-AAGGGCAGCATGTACTCATCGACA). PCR products were analyzed on 2% agarose gels. For quantitative analysis of exon skipping levels, 1 µg of RNA was reverse transcribed using the High Capacity cDNA RT Kit (Applied Biosystems, Warrington, UK) according to manufacturer's instructions. qPCR analysis was carried out using 25ng cDNA template and amplified with Taqman Gene Expression Master Mix (Applied Biosystems, Warrington, UK) on a StepOne Plus Thermocycler (Applied Biosystems, Warrington, UK). Levels of *Dmd* exon 23 skipping were determined by multiplex qPCR of FAM-labelled primers spanning Exon 20-21 (Assay Mm.PT.47.9564450, Integrated DNA Technologies, Leuven, Belgium) and HEX-labelled primers spanning Exon 23-24 (Mm.PT.47.7668824, Integrated DNA Technologies, Leuven, Belgium). The percentage of *Dmd* transcripts skipping exon 23 was determined by normalising *Dmd* exon 23-24 amplification levels to *Dmd* exon 20-21 levels. Levels of *Acvr2b* exon 5 skipping were determined by qPCR using FAM-labelled primers spanning exon 5-6 (Mm.PT.58.32079450.g, Integrated DNA Technologies, Leuven, Belgium) and normalised to a cyclophilin B housekeeping gene (Applied Biosystems, Warrington, UK).

### MTS cytotoxicity assay

The levels of cytotoxicity of P-PMOs were assessed in human hepatocytes (Huh7) cells by incubating the cells with P-PMOs at 20 µM for dual Pip6a-PMO (*Dmd*)-PMO *(Acvr2b)* and combination treatment of Pip6a-PMO *(Dmd)* (10 µM) and Pip6a-PMO (*Acvr2b*)) (10 µM). The Huh7 cells were grown to >90% confluency in DMEM/10% FCS in a 96 well plate. The P-PMOs were made up in Opti-MEM without serum and the cells treated for 4 h at 37°C. The Opti-MEM was removed and 100 µl of fresh DMEM/10% FCS was added and the cells were incubated for 20 h at 37°C, followed by addition of 20 µl of CellTiter 96® AQueous One Solution Reagent (Promega, Southampton, UK). The level of cell viability was determined in each case by measuring the absorbance at 490 nm.

### RESULTS

### Bi-specific P-PMO design and synthesis

Novel bi-specific PMO compounds were developed that involved use of standard peptide synthesis methods for synthesis of the functionalized peptide component. The PMO components were obtained using initially unmodified PMO that was then functionalized at its 3'-end with an azido group (Figure 1A) to enable "click" conjugation. Alternatively, a 3'-disulfide functionalized PMO was used to prepare a 3'-NPys-activated PMO (Figure 1B). The yield of 3'-Npys PMO was 55%, whereas the 3'-azido PMO was in lower yield of 19.5% because of the two-step RP-HPLC purification used.

The CPP chosen for the constructions was Pip6a (Figure 2A). Two different types of bi-specific compounds were designed. In the first, the two PMO oligonucleotides were each conjugated to a different end of the Pip6a peptide (Figure 2A) (designated D1) or in the second where the PMO oligonucleotides were both attached at the carboxy-terminal end of Pip6a (designated D2 and D3). In the case of click chemistry conjugation to PMO, Pip6a was synthesized with an alkyne group either at its N-terminus for D1, or at its C-terminus for D2 and D3 (Figure 2A). In D1, PMO (*Dmd*) was conjugated to the C-terminus through an amide bond and the 3'-azido-PMO (*Acvr2b*)-was conjugated at the N-terminus through a triazole bond. For bi-specific conjugate D2 the *Acvr2b* PMO was click conjugated, whereas in conjugate D3 the *Dmd* targeting PMO was click conjugated. For click conjugations at the C-terminus of Pip6a, one β-alanine (B) and one aminohexanoic acid (X) spacer residue were incorporated on either side of the Bpg alkyne derivative, whereas no spacer was used for N-terminal click conjugations (Figure 2A). Bi-specific conjugate D4 was prepared with a similar spacing to D3, but where a disulfide bond replaced the triazole bond through synthesis of Pip6a having a C-terminal X-Cys-B extension (Figure 2B). The assembly of these bi-specific conjugates required synthesis of three different derivatives of Pip6a, which were synthesized on solid phase using Fmoc peptide chemistry and purified by RP-HPLC to greater than 95% purity in yields of 50-60%.

For each construct, conjugation of the first PMO to each of the three Pip6a derivatives was carried out through amide bond formation between the C-terminal carboxylic acid group of the peptide to the secondary amine at the 3'-end of the PMO, similarly to the synthesis of Pip6a-PMO (*Dmd*) (18) (Figures 2A and B). The conjugations were carried out in solution and purifications were carried out by ion exchange HPLC. Isolated yields of P-PMO were 35 - 40% (based on the amount of starting PMO). The 3'-azido PMO was coupled to the alkyne-P-PMO using copper (I)-mediated alkyne-azide click chemistry resulting in a yield of 42% for conjugate D1 and 38% for conjugates D2 and D3 (Figure 2A). The click reaction for syntheses of D2 and D3 was sluggish at room temperature and after 6 h only a small amount of bi-specific conjugate was formed (Figure 3A). However, heating the reaction mixture to 40 °C significantly improved the reaction rate and after 30 min the reaction had proceeded to near completion as determined by ion exchange HPLC (Figure 3B). The 3'-Npys PMO (*Dmd*) was conjugated to P-PMO (*Acvr2b*) to give a disulfide bond in bi-specific conjugate D4 (Figure 2B) in a yield of 58%. Mass spectral characterizations of conjugates D1 to D4 and their intermediates are shown in Table 1.

**Table 1. Calculated and experimentally found values for masses of peptide derivatives, functionalized PMOs and conjugates. See Figures 1 and 2 for nomenclature.**

| | [M+H]⁺ | |
|---|---|---|
| | Calculated | Experimental |
| **Peptides** | | |
| Bpg-Pip6a | 3074.0 | 3075 |
| Pip6a-X-Bpg-B | 3253.5 | 3254 |
| Pi6a-X-C-B | 3239.7 | 3241 |

| **Functionalised PMO** | | |
|---|---|---|
| 3'-Azido-PMO (*Acvr2b*) 3'-Azido-PMO (*Dmd*) | 8575.4 8567.4 | 8575 8567 |
| 3'-Npys-PMO (*Dmd*) | 8663.1 | 8664 |

| **Peptide-PMO** | | |
|---|---|---|
| Bpg-Pip6a-PMO (*Dmd*) Pip6a-X-Bpg-B-PMO (*Dmd*) | 11470.8 11648.5 | 11471 11649 |
| Pip6a-X-Bpg-B-PMO (*Acvr2b*) | 11655.0 | 11656 |
| Pip6a-X-C-B-PMO *(Acvr2b)* | 11641.7 | 11642 |

| **Bi-specific conjugates** | | |
|---|---|---|
| D1 D2 | 20046.2 20223.9 | 20048 20225 |
| D3 | 20222.4 | 20224 |
| D4 | 20150.8 | 20151 |

### Evaluation of dystrophin and activin exon-skipping activities of bi-specific P-PMOs

The efficacies of the bi-specific P-PMO constructs were assessed in an initial screening step by RT-PCR using *Dmd* exon 23 skipping in H2K *mdx* cells (Figure 4A). For all conjugates tested, high levels of exon 23 skipping were found in a dose-dependent manner (with some exon 22-23 double skipping which is frequently observed in this test system). The bi-specific conjugates D2 and D3, where both PMOs are conjugated to the C-terminus of Pip6a, exhibited better exon-skipping activity than for D1 and D4. Since bi-specific conjugate D4 was less effective than D3, this suggests that there is no advantage to use of a cleavable disulfide linkage over use of stable click chemistry for addition of the second PMO. The control singly conjugated Pip6a-PMO (*Dmd*) demonstrated only slightly higher exon skipping compared to conjugates D2 and D3.

Bi-specific conjugates D2 and D3, which showed the best *Dmd* exon skipping levels, were examined further for their exon-skipping efficacies in *Acvr2b* (Figure 4B). The results for *Acvr2b* mirrored that of *Dmd* targeting. Only very slightly higher exon skipping was observed with the control singly conjugated Pip6a-PMO compared to bi-specific counterparts D2 and D3. In addition, the general level of exon 5 skipping in the *Acvr2b* gene was found to be lower than that for *Dmd.* This is potentially because disruptive exon skipping by oligonucleotides for this gene is harder to achieve than skipping of *Dmd* exon 23 and has not been fully optimized as yet. Bi-specific conjugate D3 was marginally more efficient than for D2.

### Evaluation of dystrophin and activin exon-skipping activity in mdx mice

Since both D2 and D3 conjugates demonstrated skipping activity of both genes in cells and had the highest levels of *Dmd* exon 23 skipping, they were further evaluated *in vivo* in the *mdx* mouse model of DMD. Intramuscular administration of 0.5 nmoles of either the D2, D3 bi-specific conjugates was carried out into the TA muscle of *mdx* mice and compared with a 1: 1 molar cocktail of singly conjugated Pip6a-PMOs. Analysis of splice-switching activity was carried out two weeks post-administration. Each of the CPP-PMO conjugates demonstrated robust splice-switching activity, with higher splice-switching activity evident for *Dmd* targeting compared to *Acvr2b,* as was also seen in the *in vitro* cell culture studies. Since no significant differences between the constructs could be seen following gel analysis by RT-PCR in the case of *Dmd* gene targeting (Figure 5A), quantitative analysis of splice switching was carried out using qPCR primers to determine the reduction in the level of transcripts containing the target exons. In each case 35-40% of exon 23 skipped *Dmd* transcripts were found, when normalized to *Dmd* transcripts from non-injected control muscle, with no statistically significant differences seen between P-PMO treated mice for both singly conjugated and bi-specific conjugates. Unsurprisingly, the pattern of exon skipping was also maintained for *Acvr2b* gene targeting, where there were no significant differences seen between D2 or D3 conjugates or the singly conjugated Pip6a-PMO counterpart (Figure 5B).

### Cell viability

Cell and *in vivo* toxicities of P-PMOs are known to be predominantly a function of the peptide component and are dose-dependent (39). Therefore, the cell viability of the D2 bi-specific conjugate was assessed in human hepatocytes (Huh7) cells and compared to that of Pip6a-PMO (*Dmd*) and a mixture of the two Pip6a-PMOs against the two different targets *Dmd* and *Acvr2b,* in each case using a high equimolar concentration based on total PMO. Thus 20 µM of bi-specific conjugate D2 was compared with a mixture of 10 µM each of Pip6a-PMO (*Dmd*) and Pip6a-PMO (*Acvr2b*) and the percentage of cell survival measured (Figure 6). These results showed significantly higher cell viability for the bi-specific D2 conjugate compared to a mixture of both Pip6a-PMOs for the two individual targets.

### DISCUSSION

This study has shown that it is possible to conjugate two oligonucleotides to a single CPP such that the resulting molecule is capable of efficient targeting in cells and *in vivo*, with retained potency for both cargo molecules. Cell viability is also improved relative to equivalent concentrations of CPP conjugated to a single oligonucleotide.

## Claims

1. A molecule having the structure of formula (I):
(CPP)-(S1)-(L1)-(S2)-(L2);
or the structure of formula (II):
(L1)-(S1)-(CPP)-(S2)-(L2);
wherein:
(a)
(CPP) is a cell penetrating peptide arranged in the N to C terminal direction;
(S1) and (S2) are spacer moieties which are independently present or absent;
(L1) is a first linking moiety;
(L2) is a second linking moiety;
(b) each of (L1) and (L2) is independently attached to a separate cargo molecule which comprises or consists of an oligonucleotide;
(c) each said oligonucleotide comprises a sequence which specifically hybridises to a target sequence and either:
(i) the oligonucleotide attached to (L1) is identical to the oligonucleotide attached to (L2); or
(ii) the oligonucleotide attached to (L1) and the oligonucleotide attached to (L2) each comprise a sequence which specifically hybridises to the same target sequence; or
(iii) the oligonucleotide attached to (L1) comprises a sequence which specifically hybridises to a first target sequence in an RNA molecule and the oligonucleotide attached to (L2) comprises a sequence which specifically hybridises to a second, different target sequence in the same RNA molecule; or
(iv) the oligonucleotide attached to (L1) comprises a sequence which specifically hybridises to a first target sequence in an RNA molecule, the sequence of which RNA molecule is encoded by a sequence in the genome of a cell which at least partially overlaps on the same or the opposite DNA strand with a sequence in said genome which encodes a second RNA molecule, and the oligonucleotide attached to (L2) comprises a sequence which specifically hybridises to a target sequence in said second RNA molecule.

2. A molecule according to claim 1, wherein each of (L1) and (L2) is independently selected and each is attached to its respective cargo molecule via a covalent link.

3. A molecule according to any one of the preceding claims, wherein each of (L1) and (L2) is attached to its respective cargo molecule via a link independently selected from a disulphide bond, a thioether linkage, a thiol-maleimide linkage, an amide linkage, an oxime linkage, a morpholino linkage, or a click reaction.

4. A molecule according to any one of the preceding claims, wherein (L1) is bis-Homopropargylglycine (Bpg), a constrained cyclo-octyne, or cysteine (Cys), and (L2) is any natural or non-natural amino acid, preferably beta-Alanaine (bAla).

5. A molecule according to any one of the preceding claims wherein each of (S1) and (S2) is independently a peptide of 1 to 5 amino acids in length, preferably 1 to 2 amino acids in length.

6. A molecule according to any one of the preceding claims wherein each of (S1) and (S2) independently comprises or consists of 6-aminohexanoic acid (Axh), 4-aminobutyric acid (Abu), 8-aminocaprylic acid (Acy), beta-Alanine, p-aminobenzoic acid, isonipecotic acid, alanine, glycine or arginine.

7. A molecule of formula (I) according to one of the preceding claims, wherein (S1) is a single amino acid Axh and (S2) is absent; and/or wherein (S1) is a single amino acid Axh, (S2) is absent, (L1) is Bpg or Cys, and (L2) is bAla, giving the structure (CPP)-Axh-Bpg-bAla or (CPP)-Axh-Cys-bAla.

8. A molecule of formula (II) according to any one of claims 1 to 6, wherein (S1) and (S2) are absent, (L1) is Bpg or Cys, and (L2) is the carboxy terminus of (CPP), giving the structure Bpg-(CPP) or Cys-(CPP).

9. A molecule according to any one of the preceding claims, which is capable of targeting to and/or penetrating the membrane of a mammalian cell, preferably a human cell, preferably a muscle cell, heart cell, smooth muscle cell, liver cell, lung cell, brain cell, spinal cord cell, or any other neuron.

10. A molecule according to any one of the preceding claims, wherein (CPP) is:
(a) A PIP series peptide, preferably consisting of the amino acid sequence RXRRBRRXRYQFLIRXRBRXRB (SEQ ID NO: 1) wherein X is 6-aminohexanoic acid (Ahx) and B is beta-Alanine;
(b) A peptide of formula (RXRRBR)₂XB, wherein X is 6-aminohexanoic acid (Ahx) and B is beta-Alanine (full sequence RXRRBRRXRRBRXB; SEQ ID NO: 2; also known as B peptide);
(c) Tat peptide (GRKKRRQRRRPPQ; SEQ ID NO: 3);
(d) Transportan (GWTLNSAGYLLGKINLKALAALAKKIL; SEQ ID NO: 4);
(e) Penetratin (RQIKIWFQNRRMKWKK; SEQ ID NO: 5);
(f) R6-Penetratin (RRRRRRRQIKIWFQNRRMKWKK; SEQ ID NO: 6);
(g) pVEC (LLIILRRRIRKQAHAHSK; SEQ ID NO: 7);
(h) MPG (GALFLGFLGAAGSTMGAWSQPKKKRKV; SEQ ID NO: 8)
(i) Pep1 (KETWWETWWTEWSQPKKKRKV; SEQ ID NO: 9)
(j) MAP (KLALKLALKALKAALKLA; SEQ ID NO: 10);
(k) R6W3 (RRWWRRWRR; SEQ ID NO: 11);
(l) A peptide of formula Rₙ, wherein 6<n<12;
(m) A peptide of formula (RXR)₄, wherein X is 4-aminobutyric acid (Abu), 6-aminohexanoic acid (Ahx), or 8-aminocaprylic acid (Acy), preferably Ahx;
(n) X9R wherein X is any cell or tissue-targeting sequence and 9R is nine arginine residues added C terminal to X;
(o) RVG9R (YTIWMPENPRPGTPCDIFTNSRGKRASNGGGGRRRRRRRRRR; SEQ ID NO: 12); or
(p) A peptide of formula RXRRBRRXRILFQYRXRBRXRB (SEQ ID NO: 16); wherein X is 6-aminohexanoic acid (Ahx) and B is beta-Alanine;
preferably wherein (CPP) is a PIP series peptide consisting of the amino acid sequence RXRRBRRXRYQFLIRXRBRXRB (SEQ ID NO: 1) or (CPP) is a peptide consisting of the amino acid sequence RXRRBRRXRRBRXB (SEQ ID NO: 2; B peptide), wherein X is 6-aminohexanoic acid (Ahx) and B is beta-Alanine.

11. A molecule according to any one of the preceding claims, wherein at least one said oligonucleotide comprises or consists of DNA, RNA, a 2'-O-methyl-phosphorothioate oligonucleotide, a 2'-O-methoxy-ethyl- phosphorothioate oligonucleotide, a tricyclo-oligonucleotide, a peptide nucleic acid (PNA), a phosphorodiamidate morpholino oligonucleotide (PMO), or a locked nucleic acid (LNA); and/or wherein at least one cargo molecule was functionalised to facilitate its attachment to L1 and/or L2.

12. A molecule according to any one of the preceding claims, wherein said target sequence is associated with a disease or condition, and is preferably present in a mRNA or pre-mRNA molecule, or a ribosomal RNA, a transfer RNA, a Piwi-interacting RNA, a microRNA, or a long non coding RNA (lncRNA) such as a Natural antisense transcript (NAT);
optionally wherein said disease or condition is a muscle-related disease or condition, a heart-related disease or condition, or a neurological disease or condition, or cancer;
and optionally wherein said disease or condition is Becker muscular dystrophy, Bethlem myopathy, Central core disease, Charcot-Marie-Tooth disease (CMT), Congenital muscular dystrophy (CMD), Congenital myasthenic syndromes, Congenital myotonic dystrophy, Duchenne muscular dystrophy, Emery-Dreifuss muscular dystrophy, Facioscapulohumeral muscular dystrophy (FSH), Fibre-type disproportion, Fibrodysplasia ossificans progressiva (FOP), Inclusion body myositis (IBM), Juvenile dermatomyositis, Limb girdle muscular dystrophies (LGMD), Limb girdle muscular dystrophy 1B (LGMD 1B), Limb girdle muscular dystrophy 1C (LGMD 1C), Limb girdle muscular dystrophy 2A (LGMD 2A), Limb girdle muscular dystrophy 2B (LGMD 2B), Limb girdle muscular dystrophy 21 (LGMD 21), Manifesting carriers of Duchenne muscular dystrophy, McArdle disease, Merosin-deficient congenital muscular dystrophy: MDC1A, Metabolic disorders, Minicore (multicore) myopathy, Mitochondrial myopathies, Myasthenia gravis, Myopathy, Myotonias, Myotonic dystrophy, Myotubular (centronuclear) myopathy, Nemaline myopathy, Oculopharyngeal muscular dystrophy (OPMD), Periodic paralyses, Polymyositis, dermatomyositis and sarcoid myopathy, Rigid spine syndrome, Sarcoglycanopathies: LGMD2C, LGMD2D, LGMD2E and LGMD2F, Spinal muscular atrophy (SMA), Ullrich congenital muscular dystrophy, multiple sclerosis or a related neuroinflammatory conditions (such as Acute disseminated encephalomyelitis (ADEM), Optic Neuritis (ON), Transverse Myelitis, and Neuromyelitis Optica (NMO), neuroinflammation associated with neurodegeneration, Menkes Disease, [beta]-thalassemia, frontotemporal dementia, parkinsonism, Hutchinson-Gilford Progeria Syndrome, or Ataxia-telangiectasia mutated (ATM).

13. A molecule according to any one of the preceding claims, wherein at least one said oligonucleotide comprises or consists of:
- the sequence GGCCAAACCTCGGCTTACCT (SEQ ID NO: 13) or a sequence having at least 90% sequence identity to said sequence; or
- the sequence GGCCAAACCTCGGCTTACCTGAAAT (SEQ ID NO: 14) or a sequence having at least 90% sequence identity to said sequence; or
- the sequence GCCTCGTTTCTCGGCAGCAATGAAC (SEQ ID NO: 15) or a sequence having at least 90% sequence identity to said sequence.

14. A pharmaceutical composition comprising a molecule according to any one of the preceding claims and optionally a pharmaceutically acceptable diluent, adjuvant or carrier.

15. A molecule or composition according to any one of the preceding claims for use in a method of treatment of a disease or condition, optionally wherein said disease or condition is as defined in claim 12.

## Patentansprüche

1. Molekül mit der Struktur der Formel (I):
(CPP)-(S1)-(L1)-(S2)-(L2);
oder der Struktur der Formel (II):
(L1)-(S1)-(CPP)-(S2)-(L2);
wobei:
(a)
(CPP) ein zellpenetrierendes Peptid ist, das in der terminalen Richtung von N nach C angeordnet ist;
(S1) und (S2) Spacer-Einheiten sind, die unabhängig voneinander vorhanden sind oder fehlen;
(L1) eine erste Verknüpfungseinheit ist;
(L2) eine zweite Verknüpfungseinheit ist;
(b) jedes (L1) und (L2) unabhängig an ein separates Frachtmolekül gebunden ist, das ein Oligonukleotid umfasst oder daraus besteht;
(c) jedes Oligonukleotid eine Sequenz umfasst, die spezifisch mit einer Zielsequenz hybridisiert, wobei entweder:
(i) das an (L1) gebundene Oligonukleotid mit dem an (L2) gebundenen Oligonukleotid identisch ist; oder
(ii) das an (L1) gebundene Oligonukleotid und das an (L2) gebundene Oligonukleotid jeweils eine Sequenz umfassen, die spezifisch mit dieselben Zielsequenz hybridisiert; oder
(iii) das an (L1) gebundene Oligonukleotid eine Sequenz umfasst, die spezifisch mit einer ersten Zielsequenz in einem RNA-Molekül hybridisiert und das an (L2) gebundene Oligonukleotid eine Sequenz umfasst, die spezifisch mit einer zweiten, unterschiedlichen Zielsequenz in demselben RNA-Molekül hybridisiert; oder
(iv) das an (L1) gebundene Oligonukleotid eine Sequenz umfasst, die spezifisch mit einer ersten Zielsequenz in einem RNA-Molekül hybridisiert, wobei die Sequenz des RNA-Moleküls durch eine Sequenz im Genom einer Zelle codiert wird, die zumindest teilweise auf demselben oder dem gegenüberliegenden DNA-Strang mit einer Sequenz im Genom überlappen, die ein zweites RNA-Molekül codiert, und das an (L2) gebundene Oligonukleotid eine Sequenz umfasst, die spezifisch mit einer Zielsequenz in dem zweiten RNA-Molekül hybridisiert.

2. Molekül nach Anspruch 1, wobei jedes (L1) und (L2) unabhängig ausgewählt ist und jedes über eine kovalente Verbindung an sein jeweiliges Frachtmolekül gebunden ist.

3. Molekül nach einem der vorhergehenden Ansprüche, wobei jedes (L1) und (L2) über eine Verbindung an sein jeweiliges Frachtmolekül gebunden ist, die unabhängig ausgewählt ist aus einer Disulfidbindung, einer Thioetherbindung, einer Thiol-Maleimid-Bindung, einer Amidbindung, einer Oximbindung, einer Morpholinobindung oder einer Klickreaktion.

4. Molekül nach einem der vorhergehenden Ansprüche, wobei (L1) Bishomopropargylglycin (Bpg), ein eingeschränktes Cyclooctin oder Cystein (Cys) ist und (L2) eine beliebige natürliche oder nicht natürliche Aminosäure ist, vorzugsweise Beta-Alanain (bAla).

5. Molekül nach einem der vorhergehenden Ansprüche, wobei jedes (S1) und (S2) unabhängig ein Peptid mit einer Länge von 1 bis 5 Aminosäuren, vorzugsweise einer Länge von 1 bis 2 Aminosäuren ist.

6. Molekül nach einem der vorhergehenden Ansprüche, wobei (S1) und (S2) unabhängig voneinander 6-Aminohexansäure (Axh), 4-Aminobuttersäure (Abu), 8-Aminocaprylsäure (Acy), Beta-Alanin, p-Aminobenzoesäure, Isonipecotikumsäure, Alanin, Glycin oder Arginin umfassen oder daraus bestehen.

7. Molekül der Formel (I) nach einem der vorhergehenden Ansprüche, wobei (S1) eine einzelne Aminosäure Axh ist und (S2) fehlt; und/oder wobei (S1) eine einzelne Aminosäure Axh ist, (S2) fehlt, (L1) Bpg oder Cys ist und (L2) bAla ist, was die Struktur (CPP)-Axh-Bpg-bAla oder (CPP)-Axh-Cys-bAla ergibt.

8. Molekül der Formel (II) nach einem der Ansprüche 1 bis 6, wobei (S1) und (S2) fehlen, (L1) Bpg oder Cys ist und (L2) der Carboxyterminus von (CPP) ist, was die Struktur Bpg-(CPP) oder Cys-(CPP) ergibt.

9. Molekül nach einem der vorhergehenden Ansprüche, das in der Lage ist, auf die Membran einer Säugerzelle, vorzugsweise einer menschlichen Zelle, vorzugsweise einer Muskelzelle, einer Herzzelle, einer glatten Muskelzelle, einer Leberzelle, einer Lungenzelle, einer Gehirnzelle, einer Rückenmarkszelle oder eines beliebigen anderen Neurons abzuzielen und/oder diese(s) zu durchdringen.

10. Molekül nach einem der vorhergehenden Ansprüche, wobei (CPP) Folgendes ist:
(a) ein Peptid der PIP-Serie, vorzugsweise bestehend aus der Aminosäuresequenz RXRRBRRXRYQFLIRXRBRXRB (SEQ ID NO: 1), wobei X 6-Aminohexansäure (Ahx) und B Beta-Alanin ist;
(b) ein Peptid der Formel (RXRRBR)₂XB, wobei X 6-Aminohexansäure (Ahx) und B Beta-Alanin ist (vollständige Sequenz RXRRBRRXRRBRXB; SEQ ID NO: 2; auch als B-Peptid bekannt);
(c) ein Tat-Peptid (GRKKRRQRRRPPQ; SEQ ID NO: 3);
(d) Transportan (GWTLNSAGYLLGKINLKALAALAKKIL; SEQ ID NO: 4);
(e) Penetratin (RQIKIWFQNRRMKWKK; SEQ ID NO: 5);
(f) R6-Penetratin (RRRRRRRQIKIWFQNRRMKWKK; SEQ ID NO: 6);
(g) pVEC (LLIILRRRIRKQAHAHSK; SEQ ID NO: 7);
(h) MPG (GALFLGFLGAAGSTMGAWSQPKKKRKV; SEQ ID NO: 8)
(i) Pep1 (KETWWETWWTEWSQPKKKRKV; SEQ ID NO: 9)
(j) MAP (KLALKLALKALKAALKLA; SEQ ID NO: 10);
(k) R6W3 (RRWWRRWRR; SEQ ID NO: 11);
(l) ein Peptid der Formel Rn, wobei 6 < n < 12;
(m) ein Peptid der Formel (RXR)₄, wobei X 4-Aminobuttersäure (Abu), 6-Aminohexansäure (Ahx) oder 8-Aminocaprylsäure (Acy), vorzugsweise Ahx ist;
(n) X9R, wobei X eine beliebige Zell- oder Gewebezielsequenz ist und 9R neun Argininreste ist, die C-terminal zu X hinzugefügt werden;
(o) RVG9R (YTIWMPENPRPGTPCDIFTNSRGKRASNGGGGRRRRRRRRRR; SEQ ID NO: 12) oder
(p) ein Peptid der Formel RXRRBRRXRILFQYRXRBRXRB (SEQ ID NO: 16); wobei X 6-Aminohexansäure (Ahx) und B beta-Alanin ist;
vorzugsweise wobei (CPP) ein Peptid der PIP-Serie ist, das aus der Aminosäuresequenz RXRRBRRXRYQFLIRXRBRXRB (SEQ ID NO: 1) besteht, oder (CPP) ein Peptid ist, das aus der Aminosäuresequenz RXRRBRRXRRBRXB (SEQ ID NO: 2; B-Peptid) besteht, wobei X 6-Aminohexansäure (Ahx) und B Beta-Alanin ist.

11. Molekül nach einem der vorhergehenden Ansprüche, wobei mindestens eines der Oligonukleotide Folgendes umfasst oder daraus besteht: DNA, RNA, ein 2'-O-Methylphosphorothioat-Oligonukleotid, ein 2'-O-Methoxyethylphosphorothioat-Oligonukleotid, ein Tricyclo-Oligonukleotid, eine Peptidnukleinsäure (PNA), ein Phosphordiamidat-Morpholino-Oligonukleotid (PMO) oder eine verriegelte Nukleinsäure (LNA); und/oder wobei mindestens ein Frachtmolekül funktionalisiert wurde, um seine Bindung an L1 und/oder L2 zu ermöglichen.

12. Molekül nach einem der vorhergehenden Ansprüche, wobei die Zielsequenz mit einer Krankheit oder einem Zustand assoziiert ist und vorzugsweise in Folgendem vorhanden ist: einem mRNA- oder Prä-mRNA-Molekül oder einer ribosomalen RNA, einer Transfer-RNA, einer Piwi-interagierenden RNA, einer microRNA oder einer langen nicht-kodierenden RNA (lncRNA) wie einem natürlichen Antisense-Transkript (NAT);
gegebenenfalls wobei die Krankheit oder der Zustand eine muskelbedingte Krankheit oder ein muskelbedingter Zustand, eine herzbedingte Krankheit oder ein herzbedingter Zustand oder eine neurologische Krankheit oder ein neurologischer Zustand oder Krebs ist;
und gegebenenfalls wobei die Krankheit oder der Zustand Folgendes ist: Becker-Muskeldystrophie, Bethlem-Myopathie, Zentralkernkrankheit, Charcot-Marie-Tooth-Krankheit (CMT), angeborene Muskeldystrophie (CMD), angeborene myasthenische Syndrome, angeborene myotonische Dystrophie, Duchenne-Muskeldystrophie, Emery-Dreifuss-Muskeldystrophie, fazioskapulohumerale Muskeldystrophie (FSH), faserartiges Missverhältnis, Fibrodysplasia ossificans progressiva (FOP), Einschlusskörper-Myositis (Inclusion Body Myositis, IBM), Juvenile Dermatomyositis, Muskeldystrophien des Extremitätengürtels (Limb girdle muscular dystrophies, LGMD), Muskeldystrophie des Extremitätengürtels 1B (LGMD 1B), Muskeldystrophie des Extremitätengürtels 1C (LGMD 1C), Muskeldystrophie des Extremitätengürtels 2A (LGMD 2A), Muskeldystrophie des Extremitätengürtels 2B (LGMD 2B), Muskeldystrophie des Extremitätengürtels 21 (LGMD 21), manifestierende Träger der Duchenne-Muskeldystrophie, McArdle-Krankheit, Merosin-defiziente angeborene Muskeldystrophie: MDC1A, Stoffwechselstörungen, Minicore- (Multicore-) Myopathie, Mitochondriale Myopathien, Myasthenia gravis, Myopathie, Myotonien, Myotonische Dystrophie, Myotubuläre (zentronukleäre) Myopathie, Nemaline Myopathie, okulopharyngeale Muskeldystrophie (OPMD), periodische Lähmungen, Polymyositis, Dermatomyositis und Sarkoidmyopathie, Starre-Wirbelsäule-Syndrom, Sarkoglykanopathien: LGMD2C, LGMD2D, LGMD2E und LGMD2F, spinale Muskelatrophie (SMA), angeborene Ullrich-Muskeldystrophie, Multiple Sklerose oder verwandte neuroinflammatorische Erkrankungen (wie akute disseminierte Enzephalomyelitis (ADEM), Optikusneuritis (ON), transversale Myelitis und Neuromyelitis Optica (NMO); Neuroinflammation in Verbindung mit Neurodegeneration, Menkes-Krankheit, [Beta]-Thalassämie, frontotemporale Demenz, Parkinsonismus, Hutchinson-Gilford-Progeria-Syndrom oder Ataxia-Teleangiektasie-Mutation (ATM).

13. Molekül nach einem der vorhergehenden Ansprüche, wobei mindestens eines der Oligonukleotide Folgendes umfasst oder daraus besteht:
- die Sequenz GGCCAAACCTCGGCTTACCT (SEQ ID NO: 13) oder eine Sequenz mit mindestens 90% Sequenzidentität zur Sequenz; oder
- die Sequenz GGCCAAACCTCGGCTTACCTGAAAT (SEQ ID NO: 14) oder eine Sequenz mit mindestens 90% Sequenzidentität zur Sequenz; oder
- die Sequenz GCCTCGTTTCTCGGCAGCAATGAAC (SEQ ID NO: 15) oder eine Sequenz mit mindestens 90% Sequenzidentität zur Sequenz.

14. Pharmazeutische Zusammensetzung, die ein Molekül nach einem der vorhergehenden Ansprüche und gegebenenfalls ein pharmazeutisch verträgliches Verdünnungsmittel, ein pharmazeutisch verträgliches Adjuvans oder einen pharmazeutisch verträglichen Träger umfasst.

15. Molekül oder Zusammensetzung nach einem der vorhergehenden Ansprüche zur Verwendung in einem Verfahren zur Behandlung einer Krankheit oder eines Zustands, gegebenenfalls wobei die Krankheit oder der Zustand wie in Anspruch 12 definiert ist.

## Revendications

1. Molécule ayant la structure de formule (I) :
(CPP)-(S1)-(L1)-(S2)-(L2) ; ou
la structure de formule (II) :
(L1)-(S1)-(CPP)-(S2)-(L2);
dans lequel :
(a)
(CPP) est un peptide de pénétration cellulaire agencé dans la direction N à C terminale ;
(S1) et (S2) sont des fragments d'espacement qui sont indépendamment présentes ou absentes ;
(L1) est un premier fragment de liaison ;
(L2) est un deuxième fragment de liaison ;
(b) chacun de (L1) et (L2) est indépendamment attaché à une molécule cargo séparée qui comprend ou est constituée par un oligonucléotide ;
(c) chacun desdits oligonucléotides comprend une séquence qui s'hybride spécifiquement à une séquence cible et soit :
(i) l'oligonucléotide attaché à (L1) est identique à l'oligonucléotide attaché à (L2) ; ou
(ii) l'oligonucléotide attaché à (L1) et l'oligonucléotide attaché à (L2) comprennent chacun une séquence qui s'hybride spécifiquement à la même séquence cible ; ou
(iii) l'oligonucléotide attaché à (L1) comprend une séquence qui s'hybride spécifiquement à une première séquence cible dans une molécule d'ARN et l'oligonucléotide attaché à (L2) comprend une séquence qui s'hybride spécifiquement à une deuxième séquence cible différente dans la même molécule d'ARN ; ou
(iv) l'oligonucléotide attaché à (L1) comprend une séquence qui s'hybride spécifiquement à une première séquence cible dans une molécule d'ARN, la séquence dont la molécule d'ARN est codée par une séquence dans le génome d'une cellule qui chevauche au moins partiellement sur le même brin d'ADN ou le brin d'ADN opposé avec une séquence dans ledit génome qui code pour une deuxième molécule d'ARN, et l'oligonucléotide attaché à (L2) comprend une séquence qui s'hybride spécifiquement à une séquence cible dans ladite deuxième molécule d'ARN.

2. Molécule selon la revendication 1, dans laquelle chacun de (L1) et (L2) est sélectionné indépendamment et chacun est attaché à sa molécule cargo respective via une liaison covalente.

3. Molécule selon l'une quelconque des revendications précédentes, dans laquelle chacun de (L1) et (L2) est attaché à sa molécule cargo respective via une liaison choisie indépendamment parmi une liaison disulfure, une liaison thioéther, une liaison thiol-maléimide, une liaison amide, une liaison oxime, une liaison morpholino ou une réaction de clic.

4. Molécule selon l'une quelconque des revendications précédentes, dans laquelle (L1) est la bis-homopropargylglycine (Bpg), un cyclo-octyne contraint ou la cystéine (Cys), et (L2) est tout acide aminé naturel ou non naturel, de préférence la bêta-Alanaine (bAla).

5. Molécule selon l'une quelconque des revendications précédentes, dans laquelle chacun de (S1) et (S2) est indépendamment un peptide de 1 à 5 acides aminés de longueur, de préférence 1 à 2 acides aminés de longueur.

6. Molécule selon l'une quelconque des revendications précédentes, dans laquelle chacun de (S1) et (S2) comprend indépendamment ou est constitué par un acide 6-aminohexanoïque (Axh), un acide 4-aminobutyrique (Abu), un acide 8-aminocaprylique (Acy), une bêta-alanine, un acide p-aminobenzoïque, acide isonipécotique, une alanine, une glycine ou une arginine.

7. Molécule de formule (I) selon l'une des revendications précédentes, dans laquelle (S1) est un seul acide aminé Axh et (S2) est absent ; et / ou dans laquelle (S1) est un seul acide aminé Axh, (S2) est absent, (L1) est Bpg ou Cys, et (L2) est bAla, donnant la structure (CPP)-Axh-Bpg-bAla ou (CPP)-Axh-Cys-bAla.

8. Molécule de formule (II) selon l'une quelconque des revendications 1 à 6, dans laquelle (S1) et (S2) sont absents, (L1) est Bpg ou Cys, et (L2) est l'extrémité carboxy de (CPP), donnant la structure Bpg-(CPP) ou Cys-(CPP).

9. Molécule selon l'une quelconque des revendications précédentes, qui est capable de cibler et / ou de pénétrer la membrane d'une cellule de mammifère, de préférence une cellule humaine, de préférence une cellule musculaire, une cellule cardiaque, une cellule musculaire lisse, une cellule hépatique, une cellule pulmonaire, une cellule cérébrale, une cellule de la moelle épinière ou tout autre neurone.

10. Molécule selon l'une quelconque des revendications précédentes, dans laquelle (CPP) est :
(a) un peptide de la série PIP, constitué de préférence de la séquence d'acides aminés RXRRBRRXRYQFLIRXRBRXRB (SEQ ID NO: 1) dans lequel X est l'acide 6-aminohexanoïque (Ahx) et B est la bêta-alanine ;
(b) un peptide de formule (RXRRBR)₂XB, dans lequel X est l'acide 6-aminohexanoïque (Ahx) et B est la bêta-alanine (séquence complète RXRRBRRXRRBRXB; SEQ ID NO: 2; également connu sous le nom de peptide B) ;
(c) le peptide Tat (GRKKRRQORRRPPQ; SEQ ID NO: 3) ;
(d) Transportan (GWTLNSAGYLLGKINLKALAALAKKIL; SEQ ID NO: 4) ;
(e) Pénétratine (RQOIKTIWFQNRRMKWKK; SEQ ID NO: 5) ;
(f) R6-Pénétratine (RRRRRRRQIKIWFONRRMKWKK; SEQ ID NO: 6) ;
(g) pVEC (LLITILRRRIRKQAHAHSK; SEQ ID NO: 7) ;
(h) MPG (GALFLGFLGAAGSTMGAWSQPKKKRKV; SEQ ID NO: 8) ;
(i) Pep1 (KETWWETWWTEWSQPKKKRKV; SEQ ID NO: 9) ;
(j) MAP (KLALKLALKALKAALKLA; SEQ ID NO: 10) ;
(k) R6W3 (RRWWRRWRR; SEQ ID NO: 11) ;
(l) un peptide de formule Rₙ, dans lequel 6 < n < 12 ;
(m) un peptide de formule (RXR)₄, dans lequel X est l'acide 4-aminobutyrique (Abu), l'acide 6-aminohexanoïque (Ahx) ou l'acide 8-aminocaprylique (Acy), de préférence Ahx ;
(n) X9R où X est n'importe quelle séquence de ciblage cellulaire ou tissulaire et 9R est neuf résidus arginine à C-terminaux ajoutés à X ;
(o) RVG9R (YTIWMPENPRPGTPCDIFTNSRGKRASNGGGGRRRRRRRRRR; SEQ ID NO: 12) ; ou
(p) un peptide de formule RXRRBRRXRILFQYRXRBRXRB (SEQ ID NO: 16) ; dans lequel
X est l'acide 6-aminohexanoïque (Ahx) et B est la bêta-alanine ;
de préférence dans laquelle (CPP) est un peptide de la série PIP constitué de la séquence d'acides aminés RXRRBRRXRYQFLIRXRBRXRB (SEQ ID NO: 1) ou (CPP) est un peptide constitué de la séquence d'acides aminés RXRRBRRXRRBRXB (SEQ ID NO: 2; peptide B), dans laquelle X est l'acide 6-aminohexanoïque (Ahx) et B est la bêta-alanine.

11. Molécule selon l'une quelconque des revendications précédentes, dans laquelle au moins un desdits oligonucléotides comprend ou est constitué par l'ADN, l'ARN, un oligonucléotide 2'-O-méthyl-phosphorothioate, un oligonucléotide 2'-O-méthoxy-éthyl-phosphorothioate, un tricyclo-oligonucléotide, un acide nucléique peptidique (PNA), un phosphorodiamidate morpholino oligonucléotide (PMO) ou un acide nucléique verrouillé (LNA) ; et / ou dans laquelle au moins une molécule cargo a été fonctionnalisée pour faciliter sa fixation à L1 et / ou L2.

12. Molécule selon l'une quelconque des revendications précédentes, dans laquelle ladite séquence cible est associée à une maladie ou un état, et est de préférence présente dans une molécule d'ARNm ou pré-ARNm, ou un ARN ribosomal, un ARN de transfert, un ARN interagissant à Piwi, un microARN ou un long ARN non codant (IncRNA) tel qu'un transcrit antisens naturel (NAT) ;
éventuellement dans laquelle ladite maladie ou état est une maladie ou un état lié aux muscles, une maladie ou un état lié au cœur, ou une maladie ou état neurologique, ou un cancer ;
et éventuellement dans laquelle ladite maladie ou état est la dystrophie musculaire de Becker, la myopathie de Bethlem, la myopathie du central core, la maladie de Charcot-Marie-Tooth (CMT), la dystrophie musculaire congénitale (CMD), les syndromes myasthéniques congénitaux, la dystrophie myotonique congénitale, la dystrophie musculaire de Duchenne, la dystrophie musculaire d'Emery-Dreifuss, la dystrophie musculaire facioscapulohumérale (FSH), la disproportion de type fibre, fibrodysplasie ossifiante progressive (FOP), myosite à corps d'inclusion (IBM), dermatomyosite juvénile, dystrophies musculaires des ceintures (LGMD), dystrophies musculaires des ceintures 1B (LGMD 1B), dystrophies musculaire des ceintures 1C (LGMD 1C), dystrophie musculaire des ceintures 2A (LGMD 2A), dystrophies musculaire des ceintures 2B (LGMD 2B), dystrophies musculaire des ceintures 2I (LGMD 2I), dystrophie musculaire manifeste de Duchenne, maladie de McArdle, dystrophie musculaire congénitale déficiente en mérosine : MDC1A, troubles métaboliques, myopathie minicore (multicore), myopathies mitochondriales, myasthénie grave, myopathie, myotonies, dystrophie myotonique, myopathie myotubulaire (centronucléaire), myopathie némaline, dystrophie musculaire oculopharyngée (OPMD), paralysies périodiques, polymyosite, dermatomyosite et myopathie sarcoïde, paralysies périodiques, polymyosite, dermatomyosite et myopathie sarcoïde, syndrome de la colonne vertébrale rigide, sarcoglycanopathies LGMD2C, LGMD2D, LGMD2E et LGMD2F, amyotrophie spinale, dystrophie musculaire congénitale d'Ullrich, la sclérose en plaques ou une affection neuroinflammatoire associée (comme l'encéphalomyélite aiguë disséminée (ADEM), la névrite optique (ON), la myélite transversale et la neuromyélite optique (NMO), la neuroinflammation associée à la neurodégénérescence, la maladie de Menkes, [beta]-thalassémie, démence frontotemporale, parkinsonisme, syndrome de Hutchinson-Gilford Progeria ou mutation de l'ataxie-télangiectasie (ATM).

13. Molécule selon l'une quelconque des revendications précédentes, dans laquelle au moins un desdits oligonucléotides comprend ou est constitué par :
- la séquence GGCCAAACCTCGGCTTACCT (SEQ ID NO: 13) ou une séquence ayant au moins 90% d'identité de séquence avec ladite séquence ; ou
- la séquence GGCCAAACCTCGGCTTACCTGAAAT (SEQ ID NO: 14) ou une séquence ayant au moins 90% d'identité de séquence avec ladite séquence ; ou
- la séquence GCCTCGTTTCTCGGCAGCAATGAAC (SEQ ID NO: 15) ou une séquence ayant au moins 90% d'identité de séquence avec ladite séquence.

14. Composition pharmaceutique comprenant une molécule selon l'une quelconque des revendications précédentes et éventuellement un diluant, adjuvant ou support pharmaceutiquement acceptable.

15. Molécule ou composition selon l'une quelconque des revendications précédentes à utiliser dans un procédé de traitement d'une maladie ou d'un état, éventuellement dans laquelle ladite maladie ou état est tel que défini dans la revendication 12.
